(19) Europäisches Patentamt
European Patent Office
Office européen des brevets

(11) EP 1 904 027 B1

(12) **FASCICULE DE BREVET EUROPEEN**

(45) Date de publication et mention de la délivrance du brevet:
**31.01.2018 Bulletin 2018/05**

(21) Numéro de dépôt: **06808050.6**

(22) Date de dépôt: **23.06.2006**

(51) Int Cl.:
*A61K 8/73* (2006.01) *A61K 8/81* (2006.01)
*A61K 8/89* (2006.01) *A61K 8/92* (2006.01)
*A61Q 5/02* (2006.01) *A61Q 5/12* (2006.01)

(86) Numéro de dépôt international:
**PCT/FR2006/001448**

(87) Numéro de publication internationale:
**WO 2007/017564 (15.02.2007 Gazette 2007/07)**

(54) **INGREDIENT CONCENTRE POUR LE TRAITEMENT ET/OU MODIFICATION DE SURFACES, ET SON UTILISATION DANS DES COMPOSITIONS COSMETIQUES**

KONZENTRIERTER BESTANDTEIL ZUR BEHANDLUNG UND/ODER VERÄNDERUNG VON OBERFLÄCHEN UND DESSEN VERWENDUNG IN KOSMETISCHEN ZUSAMMENSETZUNGEN

CONCENTRATED INGREDIENT FOR TREATING AND/OR MODIFYING SURFACES AND THE USE THEREOF IN COSMETIC COMPOSITIONS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.06.2005 FR 0506385**

(43) Date de publication de la demande:
**02.04.2008 Bulletin 2008/14**

(73) Titulaire: **RHODIA CHIMIE**
**93300 Aubervilliers (FR)**

(72) Inventeurs:
- **SEIGNEURIN, Aline**
  **F-78150 Le Chesnay (FR)**
- **FOUCAULT, Carole**
  **F-94220 Charenton Le Pont (FR)**
- **DRENO, Anne-Gaëlle**
  **F-93270 Sevran (FR)**

(56) Documents cités:
**EP-A- 0 674 898**
**EP-A1- 1 618 864**
**EP-A2- 0 328 355**
**WO-A-94/06403**
**WO-A-99/24004**
**WO-A-99/43777**
**WO-A1-00/14056**
**FR-A1- 2 796 392**
**US-A- 5 965 500**
**US-A1- 2003 108 504**
**US-B1- 6 383 503**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 904 027 B1

## Description

La présente invention a pour objet un ingrédient concentré pour le traitement et/ou la modification de surfaces, notamment pour le traitement et/ou la modification de la peau et/ou des cheveux. L'invention a également pour objet l'utilisation de cet ingrédient dans des compositions cosmétiques, par exemple dans des shampoings, des gels-douche ou dans des après-shampoings, destinés à être rincés ou non.

Les compositions pour le traitement et/ou la modification de surfaces, par exemple les compositions cosmétiques sont réalisées à partir d'ingrédients, mélangés, afin de procurer à la composition l'usage voulu. Les fonctions et propriétés des compositions sont donc liées aux ingrédients présents dans la composition, et/ou à leurs interactions entre eux, et/ou à leurs interactions avec la surface0 et/ou aux interactions lors de la modification d'un paramètre comme le pH, la dilution, la température.

On connaît l'utilisation de polymères cationiques, notamment des polysaccharides cationiques0 dans des compositions cosmétiques, en particulier dans les shampoings. Ces ingrédients peuvent procurer une rhéologie modifiée, et/ou une stabilisation, et/ou un conditionnement de le peau et/ou des cheveux. Des polymères cationiques utiles sont par exemple les guars cationiques ou les dérivés cellulosiques cationiques.

On connaît l'utilisation de d'agent conditionneurs dans les shampoings, notamment les polyorganosiloxanes.

On connaît l'association d'agents conditionneurs tels que des polyorganosiloxanes avec des polymères cationiques, dans des compositions cosmétiques. On a ainsi décrit la réalisation de compositions cosmétiques par ajout séparément d'un agent conditionneur, de polymères cationiques, et d'autres ingrédients. Les associations de polyorganosiloxanes et de polymères cationiques sont par exemple décrites dans le documents EP432951, EP529883, EP904045. De telles associations ont été décrites comme intéressantes en ce qui concerne la stabilité des compositions et en ce qui concerne les propriétés de conditionnement des cheveux. Il a été enseigné que le conditionnement peut être lié à un dépôt de matière sur les cheveux. Il a également été enseigné que le dépôt des agents conditionneurs peut être lié à phénomènes de formation et /ou de déstabilisation de coacervats entre le polymère cationique et des tensioactifs anioniques lors de l'application de la composition et/ou de son rinçage. US 2003/0108504 décrit des compositions de type conditionneur capillaire utiles pour contrôler le frisottis des cheveux, contenant notamment des composés siliconés non volatils de poids moléculaire allant de 100 000 à 1 500 000 et ayant une viscosité allant de 500 000 à 50 000 000 mPa.s; un composé volatil choisi parmi un hydrocarbone isoparaffine de point d'ébullition allant de 60 à 260 °C, un composé siliconé ayant de 2 à 7 atomes de silicium ou leurs mélanges; et un agent de contrôle du frisottis des cheveux. FR 2 796 392 décrit une composition nettoyante ou de rinçage comprenant au moins un composé polymère organique hydrosoluble ou hydrodispersable ayant à la fois une fonction d'interaction avec la surface à traiter et une fonction conférant à cette surface un caractère hydrophile.

WO 00/14053 décrit un monomère amphotère particulier et des polymères ou copolymères incluant un tel monomère. L'utilisation de tels polymères ou copolymères dans des formulations d'hygiène corporelle ou de détergence est également envisagée.

On cherche toujours, notamment dans les compositions cosmétiques destinées au traitement de la peau et/ou des cheveux, à optimiser certaines propriétés comme la viscosité, la transparence, le dépôt de matière (effet conditionneur), et/ou, plus généralement, à optimiser des effets cosmétiques tels la douceur, la souplesse, le démêlage, la brillance, l'aptitude au coiffage sur cheveux secs ou mouillés. Bien entendu, outre les effets apportés par les ingrédients, on cherche également des formulations faciles à préparer, faciles mettre en oeuvre, et suffisamment stables.

On cherche toujours à proposer de nouveaux ingrédients, utiles notamment pour des compositions cosmétiques, en particulier destinées à être rincées, présentant des qualités améliorées en matière de stabilité et/ou de simplification des formulations et/ou de qualités cosmétiques (mentionnées ci-dessus) et/ou de dépôt de matière (dépôt d'un polymère portant des charges cationiques ou dépôt d'autres matières comme des huiles animales, minérales, végétales ou synthétiques, par exemples de huiles silicones, ou "polyorganosiloxanes").

L'invention a pour objet de répondre aux besoins mentionnés ci-dessus en proposant un ingrédient concentré pour le traitement et/ou la modification de surfaces, tel que défini en revendication 1.

L'invention a également pour objet l'utilisation de l'ingrédient dans des compositions cosmétiques.

L'invention a également pour objet un procédé de préparation de compositions cosmétiques comprenant une étape de mélange de l'ingrédient avec d'autres produits, notamment un vecteur cosmétiquement acceptable.

L'invention a également pour objet un procédé de préparation de compositions cosmétiques comprenant une étape de préparation de l'ingrédient puis une étape de mélange de l'ingrédient avec d'autres produits, notamment un vecteur cosmétiquement acceptable.

L'invention a également pour objet un procédé de traitement et/ou de modification de surfaces, de préférence la peau et/ou les cheveux, comprenant une étapes d'application d'une composition comprenant l'ingrédient concentré, de préférence une composition cosmétique, et une étape de d'application de la composition sur la surface.

On constate que la mise en oeuvre de l'ingrédient concentré comprenant l'agent conditionneur et le polymère d'aide au dépôt, permet de modifier substantiellement les propriétés de conditionnement, notamment d'augmenter le

dépôt, d'une composition dans laquelle il est introduit, comparativement à la mise en oeuvre d'une association des produits de l'ingrédient, par introduction séparément dans la composition.

Formulation et forme de l'ingrédient

L'ingrédient concentré est sous forme liquide (fluide). Il s'agit d'un ingrédient concentré comprenant de l'eau, sous forme d'une émulsion directe comprenant des gouttelettes de l'agent conditionneur a) dispersées dans l'eau. Dans l'ingrédient concentré, la quantité d'eau en poids est inférieure à 90% en poids, de préférence inférieure à 75% en poids. Cette quantité peut être même inférieure 50% en poids, et même nulle.
Le rapport en poids entre le produit b) et le produit a) dans l'ingrédient concentré est de préférence compris entre 0,05 (5/100) et 9 (90/10), de préférence entre 0,05 et 0,5 (25/50), de préférence entre 0,075 et 0,3.
L'ingrédient comprend:

- de 10 à 75 % en poids de produit a), de préférence de 20% à 70%,
- de 0,5 à 20% en poids de produit b), de préférence de 1 à 15% en poids,
- de 0 à 15% en poids de produit c), et
- de l'eau.

On mentionne que l'ingrédient concentré peut comprendre en plus un actif destiné à produire un effet sur la peau et/ou les cheveux. Il peut par exemple s'agir d'agents anti-pelliculaires, d'agent de protection des UV, d'agents de protection de la coloration. Ces actifs peuvent être des composés organiques ou des particules minérales. Les actifs, s'ils sont compris dans l'ingrédient concentré peuvent être dispersés dans l'agent conditionneur a):

- en solution, éventuellement avec un co-solvant de l'agent et de l'actif
- en dispersion de particules solides, ou
- en émulsion dispersée sous forme de gouttelettes dans l'agent conditionneur ou dans une solution comprenant l'agent conditionneur.

L'ingrédient concentré peut ainsi être une émulsion multiple comprenant une phase aqueuse externe, une phase intermédiaire, dispersée dans la phase externe, comprenant l'agent conditionneur, et une phase interne dispersée dans la phase intermédiaire, comprenant l'actif.
L'actif peut ainsi être vectorisé par l'agent conditionneur sur la peau et/ou les cheveux.
Dans l'ingrédient concentré, la charge globale du polymère d'aide au dépôt, et sa solubilité ou stabilité, peuvent varier selon le pH. De préférence le pH est tel que le charge globale est positive ou neutre.
L'ingrédient est de préférence différent d'un ingrédient comprenant une association de polyvinylpyrrolidone (PVP) ou d'un copolymère de PVP et de MAPTAC (polyquaternum 28) et d'une silicone fluide, dans un rapport de pondéral de polymère sur silicone supérieur ou égal à 90/10.
L'ingrédient est de préférence différent d'un ingrédient comprenant une huile silicone et du succinoglycane.

Agent conditionneur a)

L'ingrédient concentré comprend au moins un agent conditionneur. Il n'est pas exclu qu'il comprenne un mélange ou une association de tels agents.
L'agent conditionneur a) est une huile non-volatile insoluble dans l'eau, choisie parmi les polyorganosiloxanes.
L'ingrédient comprend une silicone (huile silicone). On entend par silicone ou polyorganosiloxane tout composé organosiloxane, comprenant des groupes alkyle (par exemple méthyle) et/ou fonctionnalisés par des groupes différents des groupes alkyle.
Le polyorganosiloxane est avantageusement (dans les shampoings et après-shampoings en particulier) un polyorganosiloxane non volatil et non-hydrosoluble. Il présente avantageusement une viscosité comprise entre 1000 et 2000000 mPa.s, de préférence entre 5000 et 1000000 mPa.s (à 25°C). Le polyorganosiloxane peut notamment être un polydiméthylorganosiloxanesiloxane ("PDMS", dénomination INCI: dimethicone), ou un polyorganosiloxane présentant des groupes amines (par exemple de l'Amodimethicone selon la dénomination INCI), ammonium quaternaire (par exemple les silicones quaternum 1 à 10 selon la dénomination INCI), hydroxyle (terminaux ou non), polyoxyalkylène, par exemple polyoxyde d'éthylène et/ou polyoxyde de propylène (en groupes terminaux, en bloc en sein d'une chaîne de PDMS, ou en greffons), des groupes aromatiques ou plusieurs de ces groupes.
Les polyorganosiloxanes utiles dans le domaine de la cosmétique et leurs caractéristiques sont connus de l'homme du métier.
Les polyorganosiloxanes (silicones) sont de préférence présentes dans l'ingrédient concentré sous forme

d'émulsion (gouttelettes liquides de silicone dispersée dans la phase aqueuse). L'émulsion peut notamment être une émulsion dont la taille moyenne des gouttelettes est supérieure ou égale à 2 µm, ou dont la taille moyenne des gouttelettes est comprise entre 0,15 µm et 2 µm, ou dont la taille moyenne des gouttelettes est inférieure ou égale à 0,15 µm.

Les gouttelettes de l'émulsion peuvent être de taille plus ou moins importante. On peut ainsi se référer à des microémulsions, à des mini-émulsions, ou à des macro-émulsions. Dans la présente demande, le terme «émulsion» couvre notamment tous ces types d'émulsions. Sans vouloir être lié à une quelconque théorie, on précise que les microémulsions sont généralement des systèmes thermodynamiquement stables, comprenant généralement d'importantes quantités d'agents d'émulsification tels que les tensioactifs c). Les autres émulsions sont généralement des systèmes en état non-thermodynamiquement stable, conservant pendant un certain temps, en état métastable l'énergie mécanique fournie lors de l'émulsification. Ces systèmes comprennent généralement des quantités moindres d'agents d'émulsification.

Les émulsions peuvent être obtenues par mélange d'une phase externe, de préférence aqueuse, du polyorganosiloxane, du polymère d'aide au dépôt, et en général d'un agent d'émulsification, puis émulsification. On peut parler d'émulsification in situ.

La taille des gouttelettes de microémulsion peut être mesurée sur une émulsion préparée préalablement à son introduction dans la composition cosmétique, par diffusion de Lumière Dynamique (DQEL), par exemple comme décrit ci-après. L'appareillage utilisé est par exemple constitué d'un laser Spectra-Physics 2020, d'un corrélateur Brookhaven 2030 et de l'informatique associée. L'échantillon étant concentré, il est dilué dans l'eau désionisée et filtré à 0,22 µm, pour être en final à 2% en poids. Le diamètre obtenu est un diamètre apparent. Les mesures sont faites à 90° et 135° d'angle. Pour les mesures de taille, outre l'analyse classique par les cumulants, trois exploitations de la fonction d'autocorrélation sont utilisées (l'échantillonnage exponentiel ou EXPSAM décrit par le Pr. Pike, la méthode « Non Negatively Constrained Least Squares » ou NNLS et la méthode CONTIN décrite par le Pr. Provencher), qui donnent chacune une répartition de taille pondérée par l'intensité diffusée, et non pas par la masse ou le nombre. Il est tenu compte de l'indice de réfraction et de la viscosité de l'eau.

Selon un mode utile, l'ingrédient concentré est transparent. Il peut par exemple présenter une transmittance d'au moins 90%, de préférence d'au moins 95%, à une longueur d'onde de 600 nm, mesurée par exemple à l'aide d'un spectromètre Lambda 40 UV-Vis, à une concentration à 0.5% en poids dans l'eau. Dans ce cadre, la composition cosmétique dans lequel il sera utilisé peut avantageusement être transparente. Elle peut par exemple présenter une transmittance d'au moins 90%, de préférence d'au moins 95%, à une longueur d'onde de 600 nm, mesurée par exemple à l'aide d'un spectromètre Lambda 40 UV-Vis.

Selon un autre mode particulier de réalisation, l'ingrédient concentré est une émulsion dont la taille moyenne des gouttelettes est supérieure ou égale à 0,15 µm, par exemple supérieure à 0,5 µm, ou à 1 µm, ou à 2 µm, ou à 10 µm, ou à 20 µm, et de préférence inférieure à 100 µm. La taille des gouttelettes peut être mesurée sur une émulsion préparée préalablement à son introduction dans la composition cosmétique, par microscopie optique et/ou granulomètrie laser (Horiba LA-910 laser scattering analyzer). Dans ce mode de réalisation, la composition dans lequel l'ingrédient sera utilisé comprend de préférence une proportion inférieure à 10% en poids d'agent d'émulsification, par rapport au poids de polyorganosiloxane.

Parmi les silicones solubles dans l'eau de la composition, on peut citer entre autres, les diméthicones copolyols (Mirasil DMCO commercialisée par la société Rhodia Chimie).

Pour ce qui a trait aux silicones se présentant sous forme de dispersions ou émulsions insolubles dans l'eau, on peut utiliser de manière convenable, des organopolysiloxanes non hydrosolubles et non volatils parmi lesquels on peut citer les huiles, gommes ou résines polyalkylsiloxanes, polyarylsiloxanes, polyalkylarylsiloxanes, ou leurs dérivés fonctionnalisés non hydrosolubles, ou leurs mélanges, non volatils.

Lesdits organopolysiloxanes sont considérés comme non hydrosolubles et non volatils, lorsque leur solubilité dans l'eau est inférieure à 50 g/litre et leur viscosité intrinsèque d'au moins 3000 mPa.s, à 25°C.

A titre d'exemples d'organopolysiloxanes ou silicones non hydrosolubles et non volatils, on peut citer des gommes silicones comme par exemple la gomme diphényl diméthicone commercialisée par la société Rhodia Chimie, et de préférence les polydiméthylorganosiloxanes présentant une viscosité au moins égale à $6.10^5$ mPa.s, à 25°C, et de façon encore plus préférentielle, ceux d'une viscosité supérieure à $2. 10^6$ mPa.s, à 25°C, tels que la Mirasil DM 500000® commercialisée par la société Rhodia Chimie.

Selon l'invention, l'organopolysiloxane ou silicone non hydrosoluble et non volatil se trouve sous une forme dispersée au sein de l'ingrédient concentré le renfermant.

Parmi ces silicones de faible viscosité, on peut citer les silicones volatiles cycliques et les polydiméthylorganosiloxanes de faible masse.

On pourra également utiliser des dérivés de silicones fonctionnalisés comme les dérivés aminés directement sous forme d'émulsions ou à partir d'une micro-émulsion préformée. Il peut s'agir de composés connus sous le terme de silicones aminées ou de silicones hydroxylées. On cite par exemple l'huile Rhodorsil amine 21637 (Amodiméthicone) commercialisée par la société Rhodia, et le dimethiconol.

A titre de polyorganosiloxanes pouvant être utilisés, on cite notamment:

- les polyorganosiloxanes comprenant des unités $-Si(CH_2)_2O-$ et des unités $-SiY(CH_2)O-$ où Y est un groupe $-(CH_2)_3-NH(CH_2)_2-NH_2$ ou $-(CH_2)_3-NH_2$
- les polyorganosiloxanes comprenant des unités $-Si(CH_2)_2O-$ et des unités terminales - $HO-Si(CH_2)_2O-$ et/ou des unités non terminales $-Si(CH_2)(OH)O-$
- les polyorganosiloxanes comprenant des unités $-Si(CH_2)_2O-$ et des unités $-SiY(CH_2)O-$ où Y est $-L^X-Z^x-$Palc où $L^X$ est un groupe de liaison divalent, de préférence un groupe alkyle, $Z^X$ est une liaison covalente ou un groupe rotule divalent comprenant un hétéroatome, Palc est un groupe de formule $[OE]_s-[OP]_t-X'$, dans laquelle OE est un groupe de formule $-CH_2-CH_2-O-$, OP est un groupe de formule $-CH_2-CHCH_3-O-$ ou - $CHCH_3-CH_2-O-$, X' est un atome d'hydrogène ou un groupe de hydrocarboné, s est un nombre moyen supérieur à 1, et t est un nombre moyen supérieur ou égal à 0,
- les polyorganosiloxanes dont la chaîne comprend au moins un bloc comprenant des unités de formule des unités $-Si(CH_2)_2O-$ et au moins un bloc $-[OE]_s-[OP]_t-$,
- les polyorganosiloxanes comprenant des unités $-Si(CH_2)_2O-$ et/ou des unités $-Si(CH_2)RO-$ et/ou $-SiR_2O-$ et/ou $R-Si(CH_2)_2O-$ et/ou $H_3C-SiR_2O-$ et/ou $R-SiR_2O-$ où R, identique ou différent est un groupe alkyle différent d'un groupe méthyle, un groupe aryle, un groupe alkyle, un groupe alkyaryl, ou un groupe aralkyl.

Les ingrédients suivants, disponibles dans le commerce, peuvent notamment être utilisés à titre de produit a):

- Mirasil DM 500000, Rhodia (INCI: Dimethicone),
- Mirasil DME-2, Rhodia (INCI: dimethicone)
- Mirasil DME30, Rhodia (INCI: dimethicone)
- Mirasil ADM-E, Rhodia (INCI: amodimethicone)
- Dow Corning 1784 HVF, Dow Corning (INCI: Dimethiconol)
- Dow Corning 1784 HMW, Dow Corning (INCI: Divinyldimethicone/dimethicone)
- Mirasil DMCP-93, Rhodia (INCI: PEG/PPG-10/2 dimethicone)
- Parsol SLX, DSM (INCI: Polysilicone-15)
- Mirasil SM, Rhodia (INCI: Simethicone)
- Mirasil DMCO, Rhodia (INCI PEG/PPG-22/24 Dimethicone)
- Mirasil DM 100000, Rhodia (INCI: Dimethicone)
- DC200 fluid 60000, Dow Corning (INCI: Dimethicone)
- DC200 fluid 300000, Dow Corning (INCI: Dimethicone).

Polymère d'aide au dépôt b)

L'ingrédient concentré comprend au moins un polymère d'aide au dépôt. Il n'est pas exclu qu'il comprenne un mélange ou une association de tels polymères.

Le polymère d'aide au dépôt est un copolymère ampholyte de type b2) comprenant:

- 0,1 à 50% en nombre d'unités $B_{CAT}$, dérivant de la polymérisation d'au moins un composé monomère $B_{CAT}$ de formule générale I :

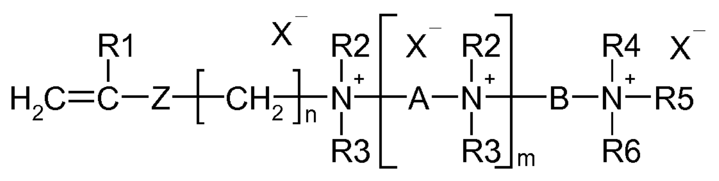

dans laquelle:

- $R_1$ est un atome d'hydrogène, un groupe méthyle ou éthyle ;
- $R_2$, $R_3$, $R_4$, $R_5$, $R_6$, identiques ou différents, sont des groupes alkyle, hydroxyalkyle ou aminoalkyle, linéaires ou ramifiés, en $C_1$-$C_6$, de préférence en $C_1$-$C_4$,
- m est un nombre entier de 0 à 10, de préférence de 0 à 2;
- n est un nombre entier de 1 à 6, de préférence 2 à 4;

- Z représente un groupe -C(O)O-, -C(O)NH- ou un atome d'oxygène;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles;
- $X^-$, identiques ou différents, représentent des contre-ions ;

- des unités $B_A$ dérivant de la polymérisation d'au moins un monomère $B_A$ hydrophile portant une fonction à caractère acide copolymérisable avec $B_A$, anionique ou susceptible d'être anionique,
- éventuellement des unités $B_N$ dérivant d'au moins un monomère $B_N$ à insaturation éthylénique, de charge neutre, copolymérisable avec $B_{CAT}$ et $B_A$, de préférence un composé monomère hydrophile à insaturation éthylénique de charge neutre portant un ou plusieurs groupes hydrophiles, copolymérisable avec $B_{CAT}$ et $B_A$,
- la quantité d'unités $B_A$ et éventuellement $B_N$ étant de 50 à 99,9 % en nombre.

Par unités ou groupes ou monomères potentiellement anioniques, on entend des unités ou groupes ou monomères dont la charge peut être neutre ou anionique suivant le pH. Par unités ou groupes ou monomères potentiellement cationiques, on entend des unités ou groupes ou monomères dont la charge peut être neutre ou cationique suivant le pH. Par unités ou groupes ou monomères zwitterioniques, on entend des unités portant simultanément deux charges.

Ces polymères peuvent être obtenus par (co)polymérisation de monomères portant les groupes cationiques ou potentiellement cationiques ou zwitterioniques. Dans la présente demande, une unité dérivant d'un monomère est entendue comme une unité telle qu'elle serait obtenue directement par polymérisation dudit monomère. Ainsi, une unité qui serait obtenue par polymérisation d'un monomère puis modification ne couvre pas l'unité issue de la polymérisation du monomère avant la modification. En revanche une telle unité couvre l'unité qui serait obtenue par un monomère conduisant après la polymérisation à une unité qui aurait la même formule que l'unité modifiée. Dans la présente demande le terme copolymère couvre les polymères comprenant deux types d'unités, trois types d'unités (on parle parfois de terpolymères) ou plus.

On mentionne que les copolymères présentant à la fois des unités cationiques ou potentiellement cationiques $B_{CAT}$ et des unités anioniques ou potentiellement anioniques $B_A$ sont souvent appelés copolymères amphotères ou ampholytes. Ils sont parfois appelés polymères zwitterioniques, à mauvais escient. Dans le présente demande un (co)polymère zwitterionique désigne un (co)polymère comprenant des unités zwitterioniques $B_Z$ et éventuellement d'autres unités.

A titre d'exemples de monomères non ioniques $B_N$ hydrophobes dont peuvent dériver les unités $B_N$ hydrophobes, on peut citer:

- les monomères vinylaromatiques tels que styrène, alpha-méthylstyrène, vinyltoluène...
- les halogénures de vinyle ou de vinylidène, comme le chlorure de vinyle, chlorure de vinylidène
- les $C_1$-$C_{12}$ alkylesters d'acides α-β monoéthyléniquement insaturés tels que les acrylates et méthacrylates de méthyle, éthyle, butyle, acrylate de 2-éthylhexyle ...
- les esters de vinyle ou d'allyle d'acides carboxyliques saturés tels que les acétates, propionates, versatates, stéarates ... de vinyle ou d'allyle
- les nitriles α-β monoéthyléniquement insaturés contenant de 3 à 12 atomes de carbone, comme l'acrylonitrile, le methacrylonitrile ...
- les α-oléfines comme l'éthylène ...
- les diènes conjugués, comme le butadiène, l'isoprène, le chloroprène.

A titre d'exemples de monomères non ioniques hydrophiles $B_N$ dont peuvent dériver les unités $B_N$ non ioniques hydrophiles, on peut mentionner:

- les hydroxyalkylesters d'acides α-β éthyléniquement insaturés comme les acrylates et méthacrylates d'hydroxyéthyle, d'hydroxypropyle, le glycérol monométhacrylate...
- les amides α-β éthyléniquement insaturés comme l'acrylamide (AM), le méthacrylamide, le N,N-diméthyl méthacrylamide, le N-méthylolacrylamide ...
- les monomères α-β éthyléniquement insaturés portant un segment polyoxyalkyléné hydrosoluble du type polyoxyde d'éthylène, comme les polyoxyde d'éthylène α-méthacrylates (BISOMER S20W, S10W, ... de LAPORTE) ou α,ω-diméthacrylates, le SIPOMER BEM de RHODIA (méthacrylate de polyoxyéthylène ω-béhényle), le SIPOMER SEM-25 de RHODIA (méthacrylate de polyoxyéthylène ω-tristyrylphényle) ...
- les monomères α-β éthyléniquement insaturés précurseurs d'unités ou de segments hydrophiles tels que l'acétate de vinyle qui, une fois polymérisés, peuvent être hydrolysés pour engendrer des unités alcool vinylique ou des

segments alcool polyvinylique,

- la vinylpyrrolidone (VP)
- les monomères $\alpha$-$\beta$ éthyléniquement insaturés de type uréido et en particulier le méthacrylamido de 2-imidazolidinone éthyle (Sipomer WAM II de RHODIA)

A titre d'exemples de monomères anioniques ou potentiellement anioniques $B_A$, dont peuvent dériver des unités $B_A$ anioniques ou potentiellement anioniques, on peut mentionner :

- des monomères possédant au moins une fonction carboxylique, comme les acides carboxyliques $\alpha$-$\beta$ éthyléniquement insaturés ou les anhydrides correspondants, tels que les acides ou anhydrides acrylique, méthacrylique, maleique, l'acide fumarique, l'acide itaconique, le N-méthacroyl alanine, le N-acryloylglycine et leurs sels hydrosolubles,
- des monomères précurseurs de fonctions carboxylates, comme l'acrylate de tertiobutyle, qui engendrent, après polymérisation, des fonctions carboxyliques par hydrolyse,
- des monomères possédant au moins une fonction sulfate ou sulfonate, comme le 2-sulfooxyethyl méthacrylate, l'acide vinylbenzène sulfonique, l'acide allyl sulfonique, le 2-acrylamido-2méthylpropane sulfonique, l'acrylate ou le méthacrylate de sulfoethyle, l'acrylate ou le méthacrylate de sulfopropyle et leurs sels hydrosolubles,
- des monomères possédant au moins une fonction phosphonate ou phosphate, comme l'acide vinylphosphonique,... les esters de phosphates éthyléniquement insaturés tels que les phosphates dérivés du méthacrylate d'hydroxyéthyle (Empicryl 6835 de RHODIA) et ceux dérivés des méthacrylates de polyoxyalkylènes et leurs sels hydrosolubles.

A titre d'exemples de monomères zwitterioniques $B_Z$ dont peuvent dériver des unités zwitterioniques $B_Z$, on peut citer:

- les monomères portant un groupe carboxybetaïne (ammonium carboxyalkyle où le groupe alkyle est éventuellement substitué par un hydroxyle),
- les monomères portant un groupe pyrridinium carboxyalkyle où le groupe alkyle est éventuellement substitué par un hydroxyle, et
- les monomères portant un groupe imidazolium carboxyalkyle où le groupe alkyle est éventuellement substitué par un hydroxyle.

La charge globale du polymère d'aide au dépôt est avantageusement positive ou nulle, au pH de l'ingrédient concentré ou au pH d'utilisation de l'ingrédient.

Le polymère d'aide au dépôt est un copolymère ampholyte de type b2) comprenant:

- 0,1 à 50% en nombre d'unités $B_{CAT}$, dérivant de la polymérisation d'au moins un composé monomère $B_{CAT}$ de formule générale I :

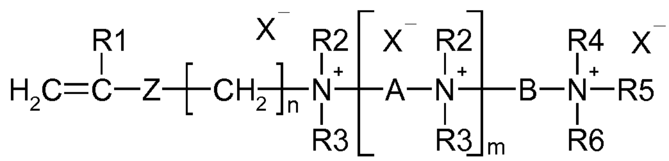

dans laquelle:

- $R_1$ est un atome d'hydrogène, un groupe méthyle ou éthyle;
- $R_2$, $R_3$, $R_4$, $R_5$, $R_6$, identiques ou différents, sont des groupes alkyle, hydroxyalkyle ou aminoalkyle, linéaires ou ramifiés, en $C_1$-$C_6$, de préférence en $C_1$-$C_4$,
- m est un nombre entier de 0 à 10, de préférence de 0 à 2;
- n est un nombre entier de 1 à 6, de préférence 2 à 4 ;
- Z représente un groupe -C(O)O-, -C(O)NH- ou un atome d'oxygène;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles ;
- X-, identiques ou différents, représentent des contre-ions;

- des unités $B_A$ dérivant de la polymérisation d'au moins un monomère $B_A$ hydrophile portant une fonction à caractère acide copolymérisable avec $B_A$, anionique ou susceptible d'être anionique,
- éventuellement des unités $B_N$ dérivant d'au moins un monomère $B_N$ à insaturation éthylénique, de charge neutre, copolymérisable avec $B_{CAT}$ et $B_A$, de préférence un composé monomère hydrophile à insaturation éthylénique de charge neutre portant un ou plusieurs groupes hydrophiles, copolymérisable avec $B_{CAT}$ et $B_A$,
- la quantité d'unités $B_A$ et éventuellement $B_N$ étant de 50 à 99,9 % en nombre.

L'ion $X^-$ est avantageusement choisi parmi halogènure, par exemple chlorure, sulfate, methyl sulfate, hydrosulfate, phosphate, citrate, forméate et acétate.

Le copolymère présente avantageusement une masse moléculaire d'au moins 1000, avantageusement d'au moins 10000 ; elle peut aller jusqu'à 20000000, avantageusement jusqu'à 10000000. Elle est de préférence comprise entre 500000 et 5000000. Sauf indications contraires, lorsqu'on parlera de masse moléculaire, il s'agira de la masse moléculaire en poids, exprimée en g/mol. Celle-ci peut être déterminée par chromatographie de perméation de gel aqueux (GPC) ou mesure de la viscosité intrinsèque dans une solution 1N de $NaNO_3$ at 30°C.

Le copolymère est de préférence statistique.

De façon préférentielle, dans la formule générale (1) du monomère $B_{CAT}$,

- Z représente C(O)O, C(O)NH ou O, tout préférentiellement C(O)NH;
- n est égal à 2 ou 3, tout particulièrement 3
- m va de 0 à 2, de préférence est égal à 0 ou 1, tout particulièrement à 0;
- B représente:

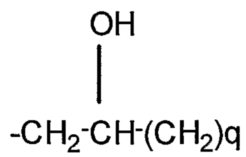

  - avec q de 1 à 4, de préférence égal à 1 ;
  - $R_1$ à $R_6$ identiques ou différents représentent un groupe méthyle ou éthyle.

Le monomère (c) préféré est le DIQUAT de formule suivante :

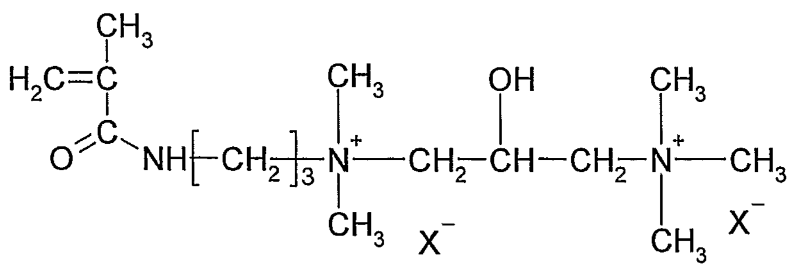

$X^-$ représentant l'ion chlorure ou méthyl sulfate.

D'autres monomères (c) particulièrement intéressants sont :

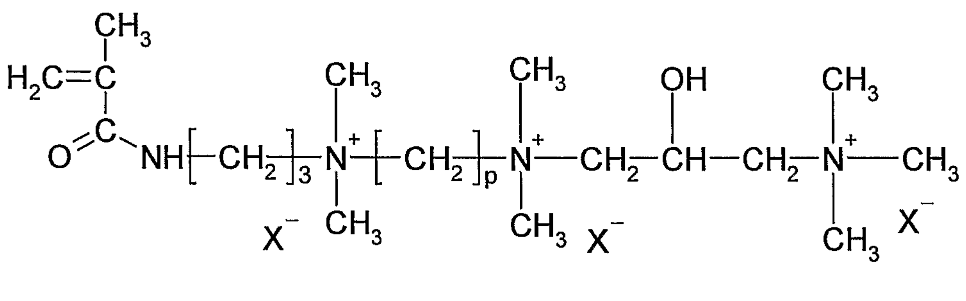

où p = 2 à 4

Les anions $X^-$ sont notamment un anion d'halogène, de préférence un anion de chlore, sulfonate, sulfate, méthyl

sulfate, hydrogénosulfate, phosphate, phosphonate, citrate, formiate et acétate.

Les monomères $B_A$ sont avantageusement des acides carboxyliques, sulfoniques, sulfuriques, phosphoniques ou phosphoriques en $C_3$-$C_8$ à insaturation monoéthylénique, leurs anhydrides et leurs sels hydrosolubles.

Parmi les monomères $B_A$ préférés, on peut citer l'acide acrylique, l'acide méthacrylique, l'acide α-éthacrylique, l'acide β,β-diméthylacrylique, l'acide méthylènemalonique, l'acide vinylacétique, l'acide allylacétique, l'acide éthylidineacétique, l'acide propylidineacétique, l'acide crotonique, l'acide maléique, l'acide fumarique, l'acide itaconique, l'acide citraconique, l'acide mésaconique, la N-méthacroyl-alanine, la N-acryloyl-hydroxy-glycine, l'acrylate de sulfopropyle, l'acrylate de sulfoéthyle, le méthacrylate de sulfoéthyle, le méthacrylate de sulfoéthyle, l'acide styrène sulfonique, l'acide vinyl sulfonique, l'acide vinylphosphonique, l'acrylate de phosphoéthyle, l'acrylate de phophonoéthyle, l'acrylate de phosphopropyle, l'acrylate de phophonopropyle, le méthacrylate de phosphoethyle, le méthacrylate de phophonoethyle, le méthacrylate de phosphopropyle, le méthacrylate de phophonopropyle, et les sels de métal alcalin et d'ammonium de ceux-ci.

Parmi les monomères $B_N$, on peut citer l'acrylamide, l'alcool vinylique, les esters d'alkyle en $C_1$-$C_4$ de l'acide acrylique et de l'acide méthacrylique, les esters d'hydroxyalkyle en $C_1$-$C_4$ de l'acide acrylique et de l'acide méthacrylique, notamment l'acrylate et le méthacrylate d'éthylène glycol et de propylène glycol, les esters polyalkoxylés de l'acide acrylique et de l'acide méthacrylique, notamment les esters de polyéthylène glycol et de polypropylène glycol, les esters de l'acide acrylique ou de l'acide méthacrylique et de polyéthylène glycol ou polypropylène glycol mono $C_1$-$C_{25}$ alkyl ethers, l'acétate de vinyle, la vinylpyrrolidone, le methylvinyléther.

Le copolymère comprend de 0,1 à 50% en nombre d'unités $B_{CAT}$, et de 50 à 99,1 % en nombre d'unités $B_A$ et éventuellement $B_N$. De préférence, le polymère comprend de 10 à 40% d'unités $B_{CAT}$ et de 60 à 90% d'unités $B_A$ et éventuellement $B_N$. Par ailleurs, le polymère ne comprend avantageusement pas d'unités $B_N$. Si le copolymère comprend des unités $B_N$, le rapport molaire entre les unités $B_A$ et le unités $B_N$ est de préférence supérieur à 1, par exemple compris entre 1 et 4.

De préférence, le polymère est tel que

- les unités $B_{CAT}$ dérivent de monomère $B_{CAT}$ de formule suivante:

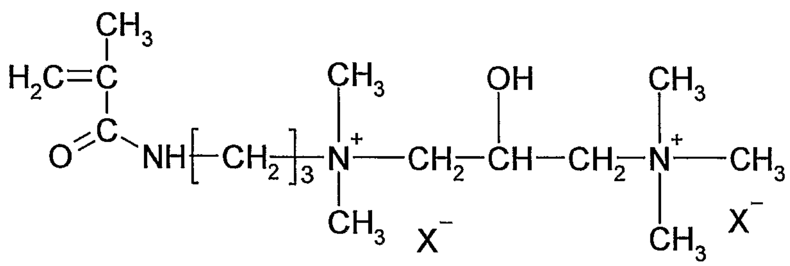

  $X^-$ représentant l'ion chlorure ou méthyl sulfate,
- les unités $B_A$ dérivent d'acide acrylique,
- le polymère ne comprend pas d'unités $B_N$,
- le rapport en nombre entre les unités $B_A$, et les unités $B_{CAT}$ est de 50/50 à 90/10.

On précise que les copolymères peuvent avoir une charge moyenne positive, négative ou nulle, au pH de l'ingrédient concentré ou au pH d'utilisation de l'ingrédient. Cette charge moyenne est définie par l'équation suivante:

$$Q = \frac{[c]X_c - [a]X_a}{[c]X_c + [a]X_a}$$

où:

- [c] est la concentration molaire en unités $B_{CAT}$,
- [a] est la concentration molaire en unités $B_A$,
- $X_C$ représente le taux de neutralisation éventuelle des unités $B_{CAT}$ (dans le cas ou les unités $B_{CAT}$ sont potentiellement cationiques); $X_C = [BH^+]/([B] + [BH^+])$,
- $X_A$ représente le taux de neutralisation éventuelle des unités $B_A$ (dans le cas ou les unités $B_A$ sont potentiellement anioniques); $X_A = [A^-]/([AH] + [A^-])$.

Un copolymère particulièrement préféré est le suivant :

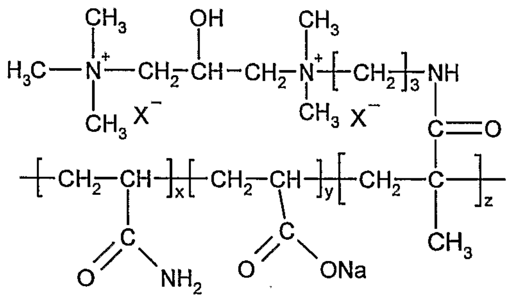

avec

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%,
- x+y+z = 100%, x, y et z représentant les % molaires de motifs dérivés respectivement d'acrylamide, acide acrylique (sel de sodium) et de DIQUAT.

D'autres polymères sont les suivants :

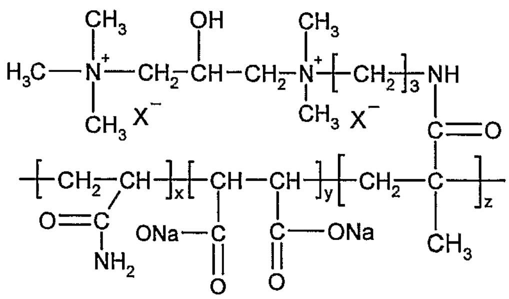

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

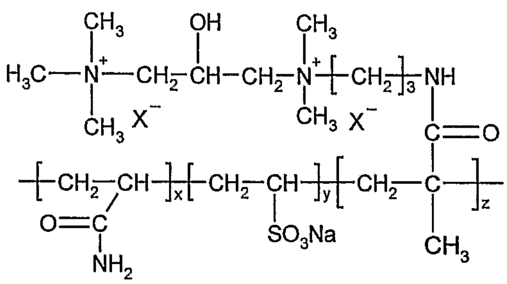

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

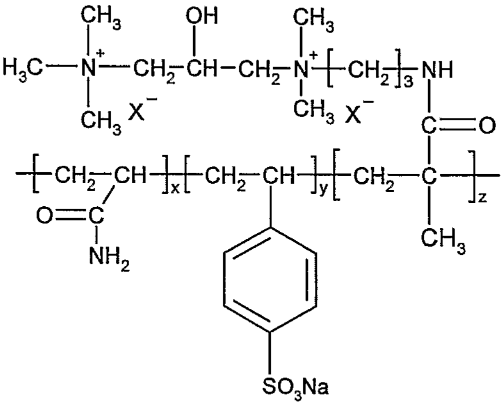

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

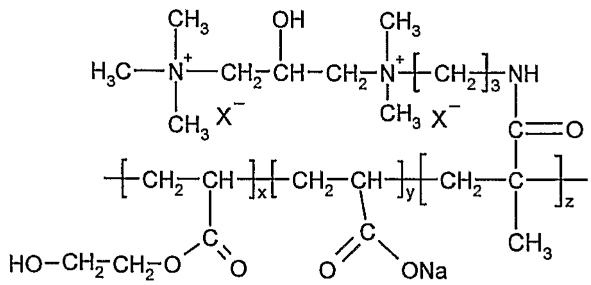

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

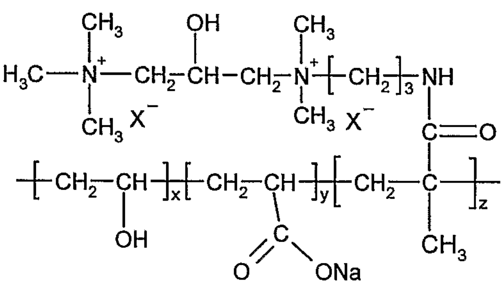

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

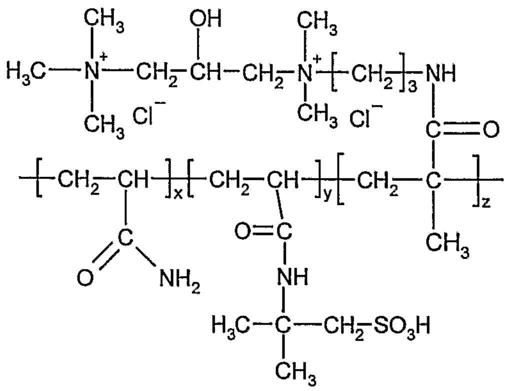

- x ayant une valeur moyenne en nombre de 0 à 50%, de préference de 0 à 30%, de préférence égale à 0
- y ayant une valeur moyenne en nombre de 10 à 95%, de préference de 50 à 70%,
- z ayant une valeur moyenne en nombre de 0,1 à 50%, de préference de 10 à 50%,
- le rapport y/z étant de manière préférée de l'ordre de 4/1 à 1/2, de préference entre 4/1 et 1/1,
- x+y est de 50 à 99,9%.

Tensioactif c)

Des tensioactifs utile, pouvant notamment servir à l'émulsification pour la réalisation d'émulsion de polyorganosiloxanes sont notamment les tensioactifs non ioniques, de préference polyalcoxylés, par exemple choisis parmi, les alcools gras alcoxylés, les triglycérides alcoxylés, les acides gras alcoxylés, les esters de sorbitan alcoxylés, les amines grasses alcoxylées, les di(phényl-1 éthyl) phénols alcoxylés, les tri(phényl-1 éthyl) phénols alcoxylés, et les alkyls phénols alcoxylés, où le nombre de motifs alcoxy, plus particulièrement oxyéthyléne et/ou oxypropyléne, est tel que la valeur de HLB est supérieure ou égale à 10.

Il est toutefois également possible d'utiliser d'autres tensioactifs, notamment ceux cités ci-dessous pour les compositions cosmétiques.

Utilisation de l'ingrédient et composition cosmétique

L'ingrédient concentré peut être utilisé dans une composition cosmétique. La composition peut être préparée par mélange de l'ingrédient concentré, préparé au préalable, avec d'autres ingrédients.

La composition cosmétique comprend ainsi généralement:

- un vecteur cosmétiquement acceptable, par exemple un vecteur aqueux, alcoolique ou hydroxyalcoolique.
- éventuellement au moins un tensioactif, et
- l'ingrédient selon l'invention, comprenant lui-même l'agent conditionneur et le polymère d'aide au dépôt.

Dans la composition cosmétique, l'agent conditionneur est avantageusement présent sous une forme physique identique à celle de l'ingrédient concentré, par exemple sous forme d'une émulsion de préférence avec une taille de gouttelettes sensiblement identique. La forme physique peut dépendre des autres ingrédients de la composition et du procédé de préparation de cette dernière. De forts cisaillements peuvent par exemple conduire à des tailles de gouttelettes inférieures.

Bien entendu, la composition cosmétique peut comprendre d'autres ingrédients. Elle peut notamment comprendre d'autres agents conditionneurs et/ou d'autres polymères d'aide au dépôt, qui peuvent être choisis parmi ceux cités plus haut.

La proportion en poids de tensioactif dans la composition est comprise entre 0 et 30%, de préférence entre 5 et 30% en poids. Le tensioactif comprend un tensioactif anionique, cationique, nonionique, ou amphotère, ou un mélange de ces tensioactifs, de préference un tensioactif anionique avec éventuellement un tensioactif amphotère.

La proportion en poids du polymère d'aide au dépôt dans la composition est de préference comprise entre 0,01 et 5%, de préference entre 0,05 et 1,5 %, de préference de 0,1 à 0,3% (en matière active).

La proportion en poids de l'agent conditionneur dans la composition peut notamment être supérieure à 1%, par exemple comprise entre 1 et 10%.

Les compositions sont de préference des compositions destinées à être rincées. Il peut s'agir par exemple d'un shampoing, d'un gel-douche ou d'un après-shampoing. Il peut néanmoins s'agir d'une composition de soin capillaire

qui n'est pas destinée à être rincée, par exemple un après shampoing destiné à ne pas être rincé, un lait démêlant, une eau démêlante, une eau de lissage, un revêtement de cuticule, un produit de soin capillaire coiffant et/ou recoiffant, un produit de protection solaire, une crème de soin, un démaquillant, un maquillage, des lingettes démaquillantes ou hydratantes, des mousses à raser, des mousses coiffantes ou fixantes.

Vecteur cosmétiquement acceptable

Il s'agit d'un vecteur d'application topique pour la peau et/ou les cheveux.

Tout vecteur cosmétiquement acceptable permettant de formuler le polymère ampholyte et d'obtenir la forme de composition cosmétique désirée, pour l'utilisation visée, peut être utilisé. Différents vecteurs cosmétiquement acceptables pour différents types de formulations sont connus de l'homme du métier.

A titre d'exemples de vecteurs cosmétiquement acceptables, on peut citer les vecteurs aqueux (comprenant de l'eau), les vecteurs alcooliques (comprenant un alcool, par exemple de l'éthanol, l'isopropanol, l'éthylène glycol ou les polyéthylène glycol), le propylène glycol, les vecteurs hydro-alcooliques (comprenant un mélange d'eau et d'un alcool par exemple de l'éthanol, l'isopropanol, l'éthylène glycol ou les polyéthylène glycol). Certaines huiles, volatiles ou non, peuvent également être utilisées. On cite par exemple les silicones fluides, tels que le cyclopentasiloxane, par exemple le Mirasil CM5 commercialisé par Rhodia.

L'homme du métier sait choisir les vecteurs adaptés aux types de formulations souhaités, et aux utilisations visées. Par exemple des vecteurs aqueux sont généralement utilisés pour des shampoings ou gels-douche. Un vecteur propylène glycol peut être utilisé des compositions sous forme de crèmes. Un vecteur cyclométhicone peut être utilisé pour des compositions de maquillage, par exemple pour des fonds de teint.

Tensioactifs de la composition cosmétique

La composition est une composition généralement aqueuse comprenant éventuellement des tensioactifs. Il peut s'agir d'un mélange de différents tensioactifs. Les tensioactifs compris dans la composition comprennent de préférence au moins un tensioactif anionique. Les tensioactifs peuvent également comprendre des tensioactifs amphotères (amphotères vrais ou zwitterioniques), des tensioactifs neutres (tensioactifs non ioniques) et/ou des tensioactifs cationiques. Les compositions comprenant au moins un tensioactif anionique et au moins un tensioactif amphotère sont particulièrement avantageuses, notamment pour des raisons de douceur. La teneur totale en tensioactifs dans la composition est comprise entre 0 et 30% en poids.

Pour des compositions destinées au traitement des cheveux, comme des shampoings, la teneur en tensioactif est avantageusement comprise entre 10 et 20% en poids. De telles compositions peuvent comprendre des sels, par exemple du chlorure de sodium ou d'ammonium, avantageusement en teneur inférieure à 3% en poids.

Pour des compositions destinées au traitement de la peau, comme des gesl-douche, la teneur en tensioactif est avantageusement comprise entre 5 et 15% en poids. De telles compositions comprennent également de préférence au moins 2% en poids de sels, par exemple du chlorure de sodium ou d'ammonium.

La proportion en poids de tensioactifs anioniques par rapport à l'ensemble des tensioactifs est de préférence supérieure à 50%, de préférence supérieure à 70%.

Pour des après-shampoings, la teneur en tensioactifs peut être inférieure à 5% en poids. Il peut s'agir de préférence de tensioactifs cationiques.

Paramètres (pH)

Le pH de la composition dépend généralement de sa destination et de son usage. Le pH est généralement compris entre 3,5 et 7,7. Il est de préférence supérieur ou égal 4,5, et plus préférablement à 5,5. Il est par exemple compris entre 5,5 et 7,5, de préférence entre 6 et 6,5. Le pH dépend évidemment des composés présents dans la composition. On peut évidemment utiliser dans la composition des agents de régulation du pH, acides ou bases, par exemple de l'acide citrique, ou de l'hydroxyde de sodium, de potassium ou d'ammonium. Pour des compositions destinés aux soins capillaires, notamment pour des après shampoings conditionnants non rincés, pouvant comprendre notamment des tensioactif cationiques, généralement en faibles quantités (moins de 5% en poids), le pH peut être relativement acide, par exemple de 3,5 à 5,5.

Natures des tensioactifs de la composition cosmétique

Les tensioactifs anioniques peuvent être choisis parmi les tensioactifs suivants:

- les alkylesters sulfonates, par exemple de formule R-CH($SO_3$M)-$CH_2$COOR', ou les alkylesters sulfates, par exemple

de formule R-CH($OSO_3M$)-$CH_2COOR'$, où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalino-terreux, par exemple sodium, ou le cation ammonium. On peut citer tout particulièrement les méthyl ester sulfonates dont le radical R est en $C_{14}$-$C_{16}$;

- les alkylbenzènesulfonates, plus particulièrement en $C_9$-$C_{20}$, les alkylsulfonates primaires ou secondaires, notamment en $C_8$-$C_{22}$, les alkylglycérol sutfonates;
- les alkylsulfates par exemple de formule $ROSO_3M$, où R représente un radical alkyle ou hydroxyalkyle en $C_{10}$-$C_{24}$, de préférence en $C_{12}$-$C_{20}$; M un cation de même définition que ci-dessus ;
- les alkyléthersulfates par exemple de formule $RO(OA)_nSO_3M$ où R représente un radical alkyle ou hydroxyalkyle en $C_{10}$-$C_{24}$, de préférence en $C_{12}$-$C_{20}$; OA représentant un groupement éthoxylé et/ou propoxylé ; M représentant un cation de même définition que ci-dessus, n variant généralement de 1 à 4, comme par exemple le lauryléthersulfate avec n = 2 ;
- les alkylamides sulfates, par exemple de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un cation de même définition que ci-dessus, ainsi que leurs dérivés polyalcoxylés (éthoxylés et/ou propoxylés) (alkylamidoether sulfates
- les sels d'acides gras saturés ou insaturés, par exemple comme ceux en $C_8$-$C_{24}$, de préférence en $C_{14}$-$C_{20}$ et d'un cation alcalino-terreux, les N-acyl N-alkyltaurates, les alkyliséthionates, les alkylsuccinamates et alkylsulfo succinates, les monoesters ou diesters de sulfosuccinates, les N-acyl sarcosinates, les polyéthoxycarboxylates ;
- les mono et di esters phosphates, par exemple de formule suivante : $(RO)_x$-P(=O)$(OM)_x$ ou R représente un radical alkyle, alkylaryle, arylalkyle, aryle, éventuellement polyalcoxylés, x et x' étant égaux à 1 ou 2, à la condition que la somme de x et x' soit égale à 3, M représentant un cation alcalino-terreux;

Les tensioactifs non ioniques peuvent être choisis parmi les tensioactifs suivants:

- les alcools gras alcoxylés ;
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
- les produits résultant de la condensation de l'oxyde d'éthylène avec un composé hydrophobe résultant de la condensation de l'oxyde de propylène avec le propylène glycol, tels les Pluronic commercialisés par BASF ;
- les produits résultant de la condensation de l'oxyde d'éthylène le composé résultant de la condensation de l'oxyde de propylène avec l'éthylènediamine, tels les Tetronic commercialisés par BASF ;
- les alkylpolyglycosides comme ceux décrits dans US 4565647;
- les amides d'acides gras par exemple en $C_8$-$C_{20}$;

Les tensioactifs amphotères (amphotères vrais comprenant un groupe ioniques et un groupe potentiellement ionique de charge opposée, ou zwitterioniques comprenant simultanément deux charges opposées) peuvent être choisis parmi les tensioactifs suivants:

- les bétaïnes de manière générale, notamment carboxybétaïnes de par exemple la lauryl bétaïne (Mirataine BB de la société Rhodia) ou l'octylbétaïne; les amidoalkylbétaïnes, comme la cocamidopropyl bétaïne (CAPB) (Mirataine BDJ de la société Rhodia Chimie) ;
- les sulfo-bétaïnes ou sultaines comme la cocamidopropyl hydroxy sultaïne (Mirataine CBS de la société Rhodia) ;
- les alkylamphoacétates et alkylamphodiacétates, comme par exemple comprenant une chaîne coco, lauryle (Miranol C2M, C32, L32 notamment, de la société Rhodia) ;
- les alkylamphopropionates ou les alkylamphodipropionates, (Miranol C2M SF) ;
- les alkyl amphohydroxypropyl sultaïnes (Miranol CS).

Les tensioactifs cationiques peuvent être choisis parmi les sels d'amines grasses primaires, secondaires ou tertiaires, éventuellement polyethoxylées, les sels d'ammonium quaternaires tels que les chlorures ou les bromures de tetraalkylammonium, d'alkylamidoalkylammonium, de trialkylbenzylammonium, de trialkylhydroxyalkylammonium, ou d'alkylpyridinium, les dérivés d'imidazoline, les oxydes d'amines à caractère cationique.

A titre d'exemples de compositions utiles, on peut citer:

- Les compositions «sodium» pour shampoings comprenant typiquement 12 à 16% en poids d'alkylethersulfate de

sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 0.5 à 2 % d'un sel (par exemple chlorure de sodium).

- Les compositions «ammonium» pour shampoings comprenant typiquement 12 à 16% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 2 % d'un sel (par exemple chlorure d'ammonium).
- Les compositions «sodium» pour gel-douche comprenant typiquement 6 à 10% en poids d'alkylethersulfate de sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 2 à 4 % d'un sel (par exemple chlorure de sodium).
- Les compositions «sodium» pour gel-douche comprenant typiquement 6 à 10% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 4 % d'un sel (par exemple chlorure d'ammonium).

Autres composés

La composition peut comprendre tout autre composé utilisé dans les compositions cosmétiques destinées à être rincées (shampoing, gel-douche, après-shampoings...), ou destinées à ne pas être rincées. Il n'est pas exclu que certains composés exercent plusieurs fonctions. De tels composés peuvent apparaître dans plusieurs sections de la présente demande.

On cite par exemple les séquestrants, les adoussissants, les modificateurs de mousse, les colorants, les agents nacrant (pearlizers), les agents hydratants, les agents antipelliculaires ou antiséborrhéiques, les agents de mise en suspension, les agents de mise en émulsion, les céramides, les pseudocéramides, les éléctrolytes, les acides gras, les esters d'acides gras, les hydroxyacides, les épaississants, les parfums, les conservateurs, les filtres solaires organiques ou minéraux, les protéines et leurs dérivés, les vitamines, des agents de stabilisation, des agents de modification de la rhéologie. Certains de ces composés sont détaillés ci-dessous.

Agents de stabilisation

La composition cosmétique peut avantageusement comprendre au moins un agent de stabilisation. On parle aussi parfois d'agents de suspension. Il n'est pas exclu que le polymère d'aide au dépôt exerce également une fonction de stabilisation.

La proportion en poids de tels agents peut être typiquement de 0,1% à 10% en poids, de préférence de 0,3% à 8% en poids, pour des polysaccharides ou d'autres agents.

A titre d'exemples d'agents de stabilisation (ou agents stabilisants), particulièrement utiles pour des compositions comprenant des polyorganosiloxanes, on peut citer:

- les polyacrylates réticulés, par exemple les polymères de type CARBOPOL ou CARBOMER commercialisés par BF Goodrich ou Noveon, ACRITAMER commercialisés par RITA ou TEGO CARBOMER commercialisés par Goldschmidt. Ces composés peuvent être typiquement présents en quantité de 0,1 à 3%, de préférence de 0,3 à 2%, en poids par rapport à la composition. On peut citer in particulier les copolymères réticulés d'acide méthacrylique et d'acrylate d'alkyle en $C_1$-$C_4$, tels que le Carbopol AQUA-SF1 de Noveon. A titre de composés commerciaux on cite: Carbopol ETD-2020 de Noveon, Carbopol 980 de Noveon, Carbomer C de Rhodia.
- les copolymères des acrylates/aminoacrylates/ itaconates PEG 20 alkyles $C_{10}$-$C_{30}$ commercialisés par National Starch sous le nom STRUCTURE PLUS. Ces composés peuvent être typiquement présents en quantité de 0,1 à 3%, de préférence de 0,3 à 2%, en poids par rapport à la composition.
- les solides insolubles formant un réseau dans la composition. Il peut s'agir de mono et/ou di-esters d'acides gras d'éthylène glycol, les acides gras étant de préférence en $C_{16}$-$C_{18}$. Il peut en particulier s'agir d'éthylène glycol distéarate (EGDS), par exemple commercialisé par Rhodia en concentré avec d'autres ingrédients sous le nom MIRASHEEN. Ce composé peut être typiquement présent en quantité de 3 à 10%, de préférence de 5 à 8% en poids par rapport à la composition. Un tel composé peut être introduit dans la composition par toute méthode connue, notamment par mélange à froid le cas échéant sous forme cristallisée, ou par mélange à chaud, avec le cas échéant une cristallisation subséquente. Il peut être introduit sous forme d'un mélange avec d'autres composés, notamment des tensioactifs. On cite notamment le distéaryle ether, l'éthylène glycol distéarate (EGDS) (INCI: Glycol distrearate), les stearates ou distearate polyethoxylés et/ou polypropoxylés, par exemples les PEG-3 distearates, PEG/PPG distearates, PEG-200 distearates, PEG-100 stearates. Des produits commerciaux pouvant être utilisés sont notamment, Mirasheen CP 820, Rhodia; Euperlan PK-3000 AM, Cognis; Euperlan PK-771 BENZ, Cognis; Genapol TS, Clariant (INCI PEG-3 distearate).

On peut citer également des agents viscosants, gelifiants ou texturants comme les copolymères acryliques anioniques de type ACULYNE commercialisés par ISP ou Rohm & Haas, par example l'Aculyne 22, les polysaccharides et leurs dérivés non cationiques tels que les dérivés de la cellulose comme l'hydroxypropylcellulose, l'hydroxyethylcellulose, la carboxyméthylcellulose, les dérivés non-ioniques de guars comme l'hydroxypropyl guar (par exemple les Jaguar HP commercialisé par Rhodia), la caroube, la gomme de tara ou de cassia, la gomme xanthane (par exemple les Rhodicare commercialisés par Rhodia), les succinoglycanes (par exemple le Rheozan commercialisé par Rhodia), les alginates, les carraghénannes, les dérivés de la chitine ou tout autre polysaccharide à fonction texturante. Ces polysaccharides et leurs dérivés peuvent être incorporés seuls ou en combinaison synergique à d'autres polysaccharides. Ces composés peuvent être typiquement présents en quantité de 0,1 à 3%, de préférence de 0,3 à 1%, en poids par rapport à la composition.

Autres ingrédients de la composition cosmétique

On peut aussi incorporer dans la composition cosmétique, sous forme de dispersions ou de solutions, des agents bactéricides ou fongicides afin d'améliorer la désinfection de la peau, comme par exemple le triclosan ; des agents anti-pelliculaires, comme notamment le zinc pyrithione ou l'octopyrox; des agents insecticides comme les pyréthroides naturels ou de synthèse.
Les compositions cosmétiques peuvent également contenir des agents pour la protection de la peau et/ou des cheveux contre les agressions du soleil et des rayons UV. Ainsi, les compositions peuvent comprendre des filtres solaires qui sont des composés chimiques absorbant fortement le rayonnement UV, comme les composés autorisés dans la directive européenne N° 76/768/CEE, ses annexes et les modifications ultérieures de cette directive.
Dans le cas où les divers éléments constitutifs de la composition cosmétique présentent une solubilité trop faible dans la composition ou lorsqu'ils se présentent sous forme solide à température ambiante, lesdits éléments constitutifs peuvent avantageusement être solubilisés dans un véhicule organique, comme des huiles minérales ou naturelles, des dérivés de silicones, des cires, ou bien encore encapsulés dans des matrices comme des polymères de type latex.
Les compositions cosmétiques faisant l'objet de l'invention peuvent également contenir des résines fixatives.
Ces résines fixatives, lorsqu'elles sont présentes, le sont généralement à des concentrations comprises entre 0,01 et 10%, préférentiellement entre 0,5 et 5%.
Les résines fixatives entrant dans les compositions cosmétiques sont plus particulièrement choisies parmi les résines suivantes :

- les copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylate de méthyle, copolymères polyvinylpyrrolidone / acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate/ acide méthacrylique, monométhyl éthers de poly(méthylvinyl éther / acide maléique), polyvinylacétates greffés sur des troncs polyoxyéthylènes (EP-A-219 048),
- les copolyesters dérivés d'acide, anhydride ou d'un diester téréphtalique et/ou isophtalique et/ou sulfoisophtalique et d'un diol, tels que :
  - les copolymères polyesters à base de motifs ethylène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
  - les oligomères polyesters sulfonés obtenus par sulfonation d'un oligomère dérivé de l'alcool allylique éthoxylé, du diméthyltéréphtalate et du 1,2 propylène diol (US-A-4 968 451) ;
  - les copolymères polyesters dérivés de diméthyltéréphtalate, d'acide isophtalique, de sulfoisophtalate de diméthyl et d'éthylène glycol (EP-A-540374)
  - les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).
  - les oligomères polyesters sulfonés obtenus par condensation de l'acide isophtalique, du sulfosuccinate de diméthyle et de diéthylène glycol (FR-A-2 236 926)
  - les copolymères polyesters à base de motifs propylène téréphtalate et polyoxyéthylène téréphtalate et terminés par des motifs éthyles, méthyles (US-A-4 711 730) ou des oligomères polyesters terminés par des groupes

alkylpolyéthoxy (US-A-4 702 857) ou des groupes anioniques sulfopolyéthoxy (US-A-4 721 580), sulfoaroyles (US-A-4 877 896) ;

- les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698) ;

• les monoamines ou polyamines éthoxylées, les polymères d'amines éthoxylées (US-A-4 597 898, EP-A-11 984).

De manière préférentielle, les résines fixatives sont choisies parmi les polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques peuvent également contenir des dérivés polymériques exerçant une fonction protectrice.

Ces dérivés polymériques peuvent être présents en quantités de l'ordre de 0,01-10%, de préférence environ 0,1-5%, et tout particulièrement de l'ordre de 0,2-3% en poids.

Ces agents peuvent notamment être choisis parmi :

• les dérivés cellulosiques non ioniques tels que les hydroxyéthers de cellulose, la méthylcellulose, l'éthylcellulose, l'hydroxypropyl méthylcellulose, l'hydroxybutyl méthylcellulose ;
• les polyvinylesters greffés sur des troncs polyalkylénés tels que les polyvinylacétates greffés sur des troncs polyoxyéthylènes (EP-A-219 048);
• les alcools polyvinyliques.

Les compositions cosmétiques faisant l'objet de l'invention peuvent aussi comprendre des agents plastifiants.

Lesdits agents, s'ils sont présents, peuvent représenter entre 0,1 à 20% de la formulation de préférence de 1 à 15%.

Parmi les agents plastifiants particulièrement utiles, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrant le calcium comme les ions citrates.

On peut également incorporer aux compositions cosmétiques faisant l'objet de l'invention des agents humectants, parmi lesquels figurent, entre autres, le glycérol, le sorbitol, l'urée, le collagène, la gélatine, l'aloe vera, l'acide hyaluronique ou des solvants volatils hydrosolubles comme l'éthanol ou le propylène glycol dont les teneurs peuvent atteindre jusqu'à 60% en poids de la composition.

Pour diminuer encore l'irritation ou l'agression du cuir chevelu, on peut aussi ajouter des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation et les dérivés de ces polycarbohydrates comme les celluloses modifiées non ioniques comme par exemple l'hydroxyéthylcellulose, ou anionique comme la carboxyméthylcellulose ; les dérivés du guar ou de la caroube comme leurs dérivés non-ioniques (par exemple hydroxypropylguar) ou les dérivés anioniques (carboxyméthylguar et carboxyméthylhydroxypropylguar).

A ces composés, on peut ajouter en association, des poudres ou des particules minérales comme du carbonate de calcium, du bicarbonate de sodium, du dihydrogénophosphate de calcium, des oxydes minéraux sous forme de poudre ou sous forme colloïdale (particules de taille inférieure ou de l'ordre de un micromètre, parfois de quelque dizaines de nanomètres) comme du dioxyde de titane, de la silice, des sels d'aluminium utilisés généralement comme antitranspirants, du kaolin, du talc, des argiles et leurs dérivés, etc..

Des agents conservateurs comme les méthyl, éthyl, propyl et butyl esters de l'acide p-hydroxybenzoïque, le benzoate de sodium, le GERMABEN® ou tout agent chimique évitant la prolifération des bactéries ou des moisissures et utilisé traditionnellement des les compositions cosmétiques, peuvent aussi être introduits dans les compositions aqueuses cosmétiques selon l'invention, généralement à hauteur de 0,01 à 3 % en poids.

La quantité de ces produits est habituellement ajustée pour éviter toute prolifération de bactéries, moisissures ou levures dans les compositions cosmétiques.

Alternativement à ces agents chimiques, on peut parfois utiliser des agents modifiant l'activité de l'eau et augmentant fortement la pression osmotique comme les carbohydrates ou des sels.

Pour protéger la peau et/ou les cheveux des agressions du soleil et des rayons UV, on peut ajouter aux compositions des filtres solaires organiques ou minéraux, par exemple des particules minérales comme l'oxyde de zinc,

le dioxyde de titane ou les oxydes de cérium sous forme de poudre ou de particules colloïdales, seuls ou en mélange. Ces poudres peuvent éventuellement être traitées en surface pour augmenter l'efficacité de leur action anti-UV ou pour faciliter leur incorporation dans les formulations cosmétiques ou pour inhiber la photoréactivité de surface. Les filtres solaires organiques peuvent notamment être introduit dans le polyorganosiloxanes, s'il est présent dans la composition.

A ces ingrédients on peut ajouter, si nécessaire, et dans le but d'augmenter le confort lors de l'utilisation de la composition par le consommateur, un ou des parfums, des agents colorants parmi lesquels on peut citer les produits décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments.

Bien que cela ne soit pas obligatoire, la composition peut aussi contenir des polymères viscosants ou gélifiants de façon à ajuster la texture de la composition, comme les polyacrylates réticulés (Carbopol commercialisés par Goodrich), déjà mentionnés ci-dessus, les dérivés non cationiques de la cellulose comme l'hydroxypropylcellulose, la carboxyméthylcellulose, les guars et leurs dérivés non ioniques, la gomme xanthane et ses dérivés, utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1-7% en poids, pour contrôler la dureté en calcium et magnésium, agents tels que :

- les sels hydrosolubles d'acides polycarboxyliques de masse moléculaire en poids de l'ordre de 2000 à 100000 g/mol, obtenus par polymérisation ou copolymérisation d'acides carboxyliques éthyléniquement insaturés tels que l'acide acrylique, l'acide ou l'anhydride maléique, l'acide fumarique, l'acide itaconique, l'acide aconitique, l'acide mésaconique, l'acide citraconique, l'acide méthylènemalonique, et tout particulièrement les polyacrylates de masse moléculaire en poids de l'ordre de 2 000 à 10 000 g/mol (US-A-3 308 067), les copolymères d'acide acrylique et d'anhydride maléique de masse moléculaire en poids de l'ordre de 5000 à 75 000 g/mol (EP-A-66 915) ;
- les polyéthylèneglycols de masse moléculaire en poids de l'ordre de 1000 à 50000 g/mol.

D'autres détails ou avantages de l'invention pourront apparaître au vu des exemples qui suivent, sans caractère limitatif.

## EXEMPLES

Pour la mise en oeuvre des exemples 1 à 6, on utilise notamment les produits suivants:

| Produit | Type | Composé |
|---|---|---|
| SLES | Tensioactif anionique | laurylethersulfate de sodium (2EO), EMPICOL ESB/3M commercialisé par Hunstman |
| CAPB | Tensioactif amphotère | Cocoamidopropylbétaïne, MIRATAINE BET-C-30 commercialisé par Rhodia |
| Sel | | Chlorure de Sodium ou Chlorure d'ammonium |
| Polymère A | | Copolymère comprenant 33% en nombre d'unités dérivées de DIQUAT et 67% en nombre d'unités dérivant d'acide acrylique, de masse moléculaire d'environ 1000 000. |
| Silicone 1 | Amodimethicone | huile ADM commercialisée par Rhodia : Rhodorsil amine oil 21637 |
| Silicone 2 | Amodimethicone | Mirasil ADM-E émulsion d'amodimethicone commercialisée par Rhodia |
| Silicone 3 | Diméthicone | Mirasil DM 500000 commercialisée par RHODIA |
| Tensioactif C | Tensioactif non ionique | Rhodasurf TR6 commercialisé par Rhodia: alcool en C13 éthoxylé 6 fois. |

### Exemple 1: Préparation d'un ingrédient concentré "Mélange 1"

On prépare un ingrédient concentré comprenant:

- 25 % en poids sec de Silicone 1, et

- 2% en poids sec de Polymère A
- 73 % d'eau distillée

Mélanger l'eau et le polymère à la pâle défloculeuse pendant 5 mns à 700 rpm. Ajouter l'huile Silicone sous agitation à la pâle défloculeuse, à 700 rpm pendant 1 minute. Agiter le mélange pendant 19 minutes.

La taille de l'émulsion finale est de 7 μm. Le pH final est de 5.3

Procédure:

Mélanger le polymère en solution aqueuse.

Exemple 2: Préparation d'un ingrédient concentré "Mélange 2"

On prépare un ingrédient concentré comprenant:

- 23,5% de silicone 1
- 0,34% d'acide acétique 100%
- 2,34% de tensioactif C (en matière active)
- 2,34% de propylène glycol
- 61,2% d'eau distillée
- 2,1% de polymère A (en matière active).

Procédure:

- Mélanger le tensioactif, le propylène glycol et l'eau. Mélange chauffé à 45-50°C.
- Sous agitation à la pale cadre (4 trous) à 200rpm et en température, introduction de l'huile silicone, très lentement et en continu dans la phase de tensioactif. En fin d'ajout, l'agitation est maintenue 2 à 15'. La taille des gouttes est supérieure à 1,5μm.
- L'émulsion ainsi obtenue est homogénéisée à l'ultraturrax à 9500rpm pendant 2': l'émulsion, est de petite taille (<1,5μm).
- A température ambiante, addition de l'acide acétique goutte par goutte sous agitation à la pale cadre à 400rpm. L'agitation est maintenue à 150rpm pendant 15': on observe une augmentation de la viscosité. Le pH final est égal à 5.5.
- à 11.1g d'émulsion, sous agitation à la pale cadre à 400rpm, introduction du polymère en solution aqueuse, goutte par goutte. La viscosité augmente après ajout de quelques gouttes de solution de polymère, apparition de grumeaux puis le mélange devient homogène.

Caractérisations

- L'émulsion est observée au microscope optique: pas de variation de la taille des gouttes, l'émulsion est stable et non floculée. La taille des gouttes est d'environ 0,8-0,9 μm.
- Stockage et suivi de la stabilité au cours du temps:
- Plusieurs échantillons sont conservés pour le suivi de la stabilité au cours du temps et placé à l'étuve à 45°C pour un vieillissement accéléré. L'émulsion est stable après 48h.

Exemple 3: Préparation de shampoings

On prépare des shampoings par mélange d'ingrédients parmi ceux mentionnés ci-dessus, avec éventuellement l'ingrédient selon l'invention de l'exemple 1.

Mode opératoire utilisant les composants séparés:

1. Mélanger l'eau et le polymère
2. Ajouter le CAPB
3. Ajouter le tensioactif anionique, puis l'émulsion de silicone
4. Ajuster le pH à 6-6,5 par ajout de d'hydroxyde de sodium ou d'acide citrique
5. Ajouter le sel

Mode opératoire utilisant l'ingrédient :

1. Mélanger l'eau et l'ingrédient
2. Ajouter le CAPB
3. Ajouter le tensioactif anionique
4. Ajuster le pH à 6-6,5 par ajout de d'hydroxyde de sodium ou d'acide citrique
5. Ajouter le sel

| | 3.1 | 3.2 (comparatif) |
|---|---|---|
| SLES (%) | 14 | 14 |
| CAPB (%) | 2 | 2 |
| NaCl (%) | 1,5 | 1,5 |
| Polymère A (%) | | 0,2 |
| Silicone 2 (% matière sèche) | | 2,5 |
| Mélange 1 Exemple 1 | Quantité telle que le shampoing comprend 0,2 % du polymère A et 2,5 % de silicone 2. | |
| Eau | Jusqu'à 100% | Jusqu'à 100% |

Evaluations

Des mesures de dépôt de silicone sur cheveux par fluorescence aux rayons X (quantification du silicium), après application du shampoing et rinçage, montrent que le shampoing de l'exemple 3.1 conditionne mieux que le shampoing de l'exemple comparatif 3.2C.

Exemple 4 : Préparation d'un ingrédient concentré "Mélange 3"

On prépare à température ambiante un ingrédient concentré comprenant:

- 65 % de silicone 3
- 1,90 % de tensioactif C
- 26,41 % d'eau distillée
- 6,69 % de polymère A (en matière active).

Procédure:

Préparation préalable d'une phase tensio-active D

Mélanger 70 % de tensioactif C et 30 % d'eau. Le mélange est chauffé à 45 °C puis refroidi à température ambiante.
Introduire la silicone 3 dans un réacteur IKA. Ajouter la phase tensioactive D. Mélanger à 400 rpm à la pâle ancre pendant 15 minutes.
Au moyen d'un moteur d'agitation classique, diluer lentement l'émulsion avec le polymère A, préparé en solution aqueuse. Agiter à la pâle cadre à 100 rpm pendant la dilution. Homogénéiser 5 minutes.

Caractérisations

- L'émulsion est observée au microscope optique et au granulomètre HORIBA. La taille des gouttes est d'environ 0,8-1.0 μm.
- le pH de l'ingrédient est de 3.1
- la viscosité Brookfield mesurée à 21 °C, 10 rpm (mobile 6) est comprise entre 45 000 et 50 000 mPa.s
- Stockage et suivi de la stabilité au cours du temps:
- Plusieurs échantillons sont conservés pour le suivi de la stabilité au cours du temps et placé à l'étuve à 45°C pour un vieillissement accéléré. L'émulsion est stable après 48h.

Exemple 5 : Préparation d'un ingrédient concentré "Mélange 4"

On prépare à température ambiante un ingrédient concentré comprenant:

- 50 % de silicone 3
- 1,46 % de tensioactif C
- 26.41 % d'eau distillée
- 10 % de polymère A (en matière active).

Procédure:

Préparation préalable d'une phase tensio-active D

Mélanger 70 % de tensioactif C et 30 % d'eau. Le mélange est chauffé à 45 °C puis refroidi à température ambiante.

Introduire la silicone 3 dans un réacteur IKA. Ajouter la phase tensioactive D. Mélanger à 400 rpm à la pâle ancre pendant 15 minutes.

Au moyen d'un moteur d'agitation classique, diluer lentement l'émulsion avec le polymère A, préparé en solution aqueuse. Agiter à la pâle cadre à 100 rpm pendant la dilution. Homogénéiser 5 minutes.

Exemple 6 : Préparation d'un ingrédient concentré "Mélange 5"

On prépare à température ambiante un ingrédient concentré comprenant:

- 45 % de silicone 3
- 2.82 % de tensioactif laureth-7
- 9 % de polymère A (en matière active).
- eau distillée jusqu'à 100%.

Procédure:

La procédure est identique aux procédures mentionnées ci-dessus, le tensioactif C étant remplacé par le laureth-7, les vitesse et durée d'agitation étant ajustées de manière à obtenir la granulométrie ci-dessous.

Caractérisations

- L'émulsion est observée au microscope optique et au granulomètre HORIBA. La taille des gouttes est d'environ 0,6 μm.
- le pH de l'ingrédient est de 2.9
- la viscosité Brookfield (appareil Brookfield DV1) mesurée à 21°C, 10 rpm (mobile 4) est de 8200 cP
- L'émulsion est stable.

**Exemple 7: Formulation shampooing avec polymère A / Dimethicone / CARBOPOL AQUA SF1**

Composition (matière active):

- 14% SLES
- 2% CAPB
- 0,1% NaCl
- 0,2% Polymère A*
- 1% Diméthicone*
- 1,5% CARBOPOL SF1

* introduits sous forme du mélange 5

Matières premières utilisées :

✓ Nom commercial Carbopol Aqua SF1, Nom INCI Acrylates copolymer, 30% ma, Fournisseur NOVEON
✓ SLES : Nom commercial Empicol ESB 3M, Nom INCI Sodium Lauryl Ether Sulfate, 27% ma, Fournisseur HUNSTMAN
✓ CAPB : Nom commercial Mirataine BETC30, Nom INCI Cocamidopropylbetaine, 30% ma, Fournisseur RHODIA
✓ Mélange 5: 5 ; 54% ma
✓ Kathon CG, Fournisseur SEPPIC
✓ NaCl, Fournisseur PROLABO

Les masses des matières premières sont pesées sur une balance avec une précision de 0,01 g et/ou sur une balance de précision à 0,001 g.

A. PREPARATION PHASE 1

1/Préparation du mélange eau- épaississant
dans un becher

- pesée masse de Carbopol Aqua SF1 : 5,0 g
- pesée et ajout sur la solution liquide de 10 g d'eau distillée

mélanger à la pâle cadre 150 rpm pendant 5 minutes.
2/ Ajout du tensio-actif anionique

- pesée masse SLES : 51,9 g
- ajouter le SLES au mélange sous agitation à la pâle cadre 150 rpm, laisser tourner pendant 5 minutes.

3/ Structuration du mélange

- sous agitation faible (50 rpm), ajouter de la soude pour atteindre pH 6,6.

Laisser bien homogénéiser entre chaque ajout de soude.
4/ Ajout du tensio-actif amphotère

- pesée masse de CAPB : 6,7 g
- ajouter la CAPB à la phase sous agitation à la pâle cadre 50 rpm, et agiter le temps nécessaire à l'homogénéisation.

B. PREPARATION PHASE 2

1/Dissolution du blend dans l'eau
dans un becher :

- pesée sur balance de précision à 0,001 g, masse de mélange 5: 2,24 g
- pesée et ajout de 10 g eau distillée

agitation au barreau magnétique jusqu'à dissolution du polymère dans l'eau.
2/ ajout de sel

- pesée sur balance de précision à 0,001 g, masse de sel : 0,10 g
- dispersion sur le mélange

mélanger pendant 15 minutes
3/ ajustement du pH

- ajout de soude goutte à goutte pour atteindre zone de pH 5,5-6,5.

laisser agiter pendant 5 minutes.

C. AJOUT PHASE 2 SUR PHASE 1

sous agitation faible à la pâle cadre (50 rpm), ajouter doucement la phase 2 sur le mélange eau-SLES-CAPB-CARBOPOL AQUA SF1.
Laisser agiter jusqu'à homogénéisation.

D. AJOUT DU CONSERVATEUR

- Incorporer 2 gouttes de Kathon CG

mélanger à la pâle cadre 150 rpm pendant 15 minutes.

E. AJUSTEMENT à pH 5-5,5 avec de l'acide citrique dilué

F. RAJOUT EAU QSP 100 g

Caractérisation de la formulation :

- pH : 5,5
- viscosité sur Brookfield DV-I +, T°C=21°C, mobile 4, série RV, vitesse 10 rpm : mesure après 1 min 5 040 mPa.s
- Stabilité statifaisante

**Exemple 8: Formulation shampooing avec polymère A / Dimethicone / CARBOPOL ETD2020**

Composition (matière active):

- 14% SLES
- 2% CAPB
- 1 % CARBOPOL ETD2020
- 0,2% Polymère A*
- 1 % Diméthicone*

* introduits sous forme du mélange 5

Matières premières utilisées :

✓ Nom commercial Carbopol ETD2020, Nom INCI Acrylates /C10-30 Alkyl Acrylate Cross-Polymer, 100% ma Fournisseur NOVEON
✓ SLES : Nom commercial Empicol ESB 3M, Nom INCI Sodium Lauryl Ether Sulfate, 27% ma, Fournisseur HUNSTMAN
✓ CAPB : Nom commercial Mirataine BETC30, Nom INCI Cocamidopropylbetaine, 30% ma, Fournisseur RHODIA
✓ Mélange 5: 5 ; 54% ma
✓ Kathon CG, Fournisseur SEPPIC
✓ NaCl, Fournisseur PROLABO

Les masses des matières premières sont pesées sur une balance avec une précision de 0,01 g et/ou sur une balance de précision à 0,001 g.

A. PREPARATION PHASE 1

1/Préparation du mélange eau- épaississant
dans un becher :

- pesée 30 g d'eau distillée
- pesée masse de Carbopol ETD2020 : 1 g
- créer un vortex avec une pâle défloculeuse, disperser sur l'eau la poudre
- agiter pendant 30 minutes
- sous agitation faible avec une pâle cadre (50 rpm), ajouter de la soude à 50 % pour atteindre zone de pH 3-4

2/ Ajout du tensio-actif anionique

- pesée masse de SLES : 51,85 g
- ajouter le SLES tout doucement sous agitation à la pâle cadre 50 rpm, laisser l'agitation pendant 30 minutes.

3/ Ajout du tensio-actif amphotère

- pesée masse de CAPB : 6 ,7 g
- ajouter la CAPB sous agitation à la pâle cadre 150 rpm, mélanger le temps nécessaire à l'homogénéisation.

4/ Structuration du mélange

- sous agitation faible (50 rpm), ajouter de la soude pour atteindre pH 5,5-6.

Laisser bien homogénéiser entre chaque ajout de soude.

B. PREPARATION PHASE 2

1/Dissolution du blend dans l'eau
dans un becher :

- pesée sur balance de précision à 0,001 g, masse du mélange 5 : 2,24 g
- pesée et ajout de 5 g eau distillée

agitation au barreau magnétique jusqu'à dissolution du polymère dans l'eau.
2/ ajustement du pH

- ajout de soude à 50 % goutte à goutte pour atteindre zone de pH 5,5-6,5.

Laisser agiter pendant 5 minutes.

C. AJOUT PHASE 2 SUR PHASE 1

sous agitation faible à la pâle cadre (50 rpm), ajouter doucement la phase 2 sur le mélange eau-SLES-CAPB-CARBOPOL ETD2020.
Laisser agiter jusqu'à homogénéisation.

D. AJOUT DU CONSERVATEUR

- Incorporer 2 gouttes de Kathon CG.
  mélanger à la pâle cadre 150 rpm pendant 15 minutes.

E. AJUSTEMENT à pH 5-5,5 avec de l'acide citrique dilué

F. RAJOUT EAU QSP 100 g

Caractérisation de la formulation :

- pH : 5
- viscosité sur Brookfield DV-I +, T°C=21°C, mobile 4, série RV, vitesse 10 rpm : mesure après 1 min 3 540 mPa.s
- Stabilité statifaisante

**Exemple 9**

On prépare les produits dont les compostions sont mentionnées ci-dessous.

Les matières premières utilisées sont identifiées par les dénominations INCI et/ou par les références commerciales. Les quantités indiquées s'entendent en matière active.

Le polymère A et l'émulsion de dimethicone sont introduits sous forme du mélange 5.

Shampooing et/ou gel-douche

| | 1 | 2 | 3 | 4 | 5 | 6 | 7 | 8 |
|---|---|---|---|---|---|---|---|---|
| Sodium Lauryl Ether Sulfate (2OE) Empicol® ESB-3M (Huntsman) | 14% | 14% | 10% | 10% | 14% | 14% | 10% | 12% |
| Cocamidopropyl Betaine Mirataine® BET C-30 (Rhodia) | 2% | 2% | 2% | 2% | 2% | 2% | 2% | 3% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,3% | 0,2% | 0,3% | 0,2% | 0,3% | 0,2% | 0,3% |
| Chlorure de Sodium | 0% | 0% | 0% | 0% | 0,1% | 0,5% | 0,1% | 0% |
| Hydroxyde de sodium | 0,2% | 0,2% | 0,2% | 0,2% | 0,2% | 0,2% | 0,2% | 0,2% |
| Acrylate/$C_{10-30}$ Alkyl Acrylate Crosspolymer Carbopol® ETD-2020 (Noveon) | 1% | 1% | 1% | 1% | - | - | - | - |
| Acrylate Copolymer Carbopol® Aqua SF-1 (Noveon) | - | - | - | - | 1,5% | 1,5% | 1,5% | - |
| Carbomer Carbopol® 980 (Noveon) | - | - | - | - | - | - | - | 1,2% |
| Glycerine | - | 1% | - | 1% | - | 0,5% | - | 0,5% |
| Glycol Distearate, Laureth-4, Cocamidopropyl Betaine Euperlan® PK-3000 AM (Cognis) | - | - | 1,5% | - | - | 1,5% | - | 1,5% |
| Glycol Distearate, Laureth-7, Sodium Cocoamphoacetate, Cocamidopropyl Betaine, Sodium Laureth Sulfate Mirasheen® CP-820/G (Rhodia) | - | - | - | 2% | - | - | 2% | - |
| Acide citrique | qs | qs | qs | qs | qs | qs | qs | qs |
| Parfum, Conservateurs | qs | qs | qs | qs | qs | qs | qs | qs |
| Eau demineralise | qsp 100% | qsp 100% | qsp 100% | qsp 100% | qsp 100% | qsp 100% | qsp 100% | qsp 100% |

Shampooing et/ou gel-douch

| | 9 | 10 | 11 | 12 | 13 | 14 | 15 | 16 |
|---|---|---|---|---|---|---|---|---|
| Sodium Lauryl Ether Sulfate (2OE) Empicol® ESB-3M (Huntsman) | 14% | 11% | 10% | 10% | 12% | 12% | 10% | 10% |
| Coco Betaine Mirataine® BB-FLA (Rhodia) | 2% | - | 3% | - | - | 4% | 3% | - |
| Disodium Cocoamphodiacetate Miranol® C2M Conc NP (Rhodia) | - | 3% | - | 3% | 4% | - | - | 2% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | | | 0,5% | 0,5% | | | | 1,8% |
| Hydroxyde de sodium | - | - | - | - | - | - | 0,5% | - |
| PEG-200 Hydrogenated Glyceryl Palmate Rewoderm® LI 520-70 (Degussa) | 0,7% | 1,2% | - | - | 0,5% | - | - | - |
| PEG-150 Distearate Rewopal® PEG-6000 DS (Degussa) | - | - | - | - | - | 1% | 0,4% | - |
| Xanthan gum Rhodicare® XC (Rhodia) | - | - | 0,6% | 0,6% | 0,6% | - | - | - |
| Hydroxyethyl cellulose Natrosol® 250-HHR HEC (Aqualon) | - | - | - | - | - | - | 1% | - |
| Hydroxypropyl Guar Jaguar® HP-105 (Rhodia) | - | - | - | - | - | - | - | 0,8% |
| Propylene Glycol | 0,3% | 0,3% | - | - | - | 0,4% | 0,3% | 0,3% |
| Glycol Distearate, Laureth-4, Cocamidopropyl Betaine Euperlan® PK-3000 AM (Cognis) | 1,5% | - | 1,5% | - | - | 1,5% | - | 1,5% |
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel-douche

| | 17 | 18 | 19 | 20 | 21 | 22 | 23 | 24 |
|---|---|---|---|---|---|---|---|---|
| Sodium Lauryl Ether Sulfate (2OE) Empicol® ESB-3M (Huntsman) | 14% | 11% | 10% | 10% | 12% | 12% | 10% | 10% |
| Disodium Cocoamphodiacetate Miranol® C2M Conc NP (Rhodia) | 2% | 3% | 2% | 3% | 4% | 2% | 2% | 2% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | 0,2% | 0,15% | 0,1% | 0,15% | 0,15% | 0,15% | 0,15% | 0,15% |
| Hydroxyde de sodium | 0,2% | 0,2% | 0,2% | 0,2% | 0,2% | 0,2% | 0,2% | 0,2% |
| Acrylate/$C_{10-30}$ Alkyl Acrylate Crosspolymer Carbopol® ETD-2020 (Noveon) | 1% | 1% | 1% | 1% | 1% | 1% | 1% | 1% |
| Guar Hydroxypropyltrimonium Chloride Jaguar® C-13S (Rhodia) | 0,1% | - | - | - | - | - | - | - |
| Guar Hydroxypropyltrimonium Chloride Jaguar® Excel (Rhodia) | - | 0,1% | - | 0,1% | - | - | - | - |
| Hydroxypropyl Guar Hydroxypropyltrimonium Chloride Jaguar® C-162 (Rhodia) | - | - | 0,3% | - | - | - | - | - |
| Polyquaternium-10 Ucare Polymer® JR-400 (Amerchol) | - | - | - | 0,1% | 0,3% | - | - | - |
| Polyquaternium-7 Merquat® 550 (Nalco) | - | - | - | - | - | 0,3% | - | - |
| Polyquaternium-11 Mirapol® PQ-11 (Rhodia) | - | - | - | - | - | - | 0,05% | - |
| Polyquaternium-22 Merquat® 280 (Nalco) | - | - | - | - | - | - | - | 0,1% |
| Glycol Distearate, Laureth-4, Cocamidopropyl Betaine | 1,5% | - | - | - | 1,5% | - | 1,5% | 1,5% |

| | | | | | | | | |
|---|---|---|---|---|---|---|---|---|
| Euperlan® PK-3000 AM (Cognis) | | | | | | | | |
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel douche

| | 25 | 26 | 27 | 28 | 29 | 30 | 31 | 32 |
|---|---|---|---|---|---|---|---|---|
| Tensioactif anionique : Sodium Lauryl Ether Sulfate (2OE) Empicol® ESB-3M (Huntsman) | 14% | 11% | 10% | 10% | 12% | 12% | 10% | 10% |
| Cocamidopropyl Betaine Mirataine® BET C-30 (Rhodia) | 2% | - | 3% | - | - | 4% | 3% | 3% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | 0,1% | 0,1% | 0,1% | - | 0,1% | - | - | - |
| Acrylate Copolymer Carbopol® Aqua SF-1 (Noveon) | 1,5% | 1,5% | 1,5% | 1,5% | 1,5% | 1,5% | 1,5% | 1,5% |
| Hydroxyde de sodium | 0,2% | 0,2% | 0,2% | 0,2% | 0,2% | 0,2% | 0,2% | 0,2% |
| Guar Hydroxypropyltrimonium Chloride Jaguar® C-13S (Rhodia) | 0,1% | - | - | 0,1% | - | - | - | - |
| Polyquaternium-10 Ucare Polymer® JR-400 (Amerchol) | - | 0,3% | - | - | - | - | - | - |
| Polyquaternium-39 Merquat® Plus 3330 (Nalco) | - | - | 0,3% | - | - | - | - | - |
| Polyquaternium-44 Luviquat® UltraCare (BASF) | - | - | - | 0,1% | 0,1% | - | - | - |
| Polyquaternium-67 SoftCAT® Polymer SL (Amerchol) | - | - | - | - | - | 0,2% | - | - |
| Polymethacrylamidopropyltrimonium Chloride Polycare 133 (Rhodia) | - | - | - | - | - | - | 0,2% | |
| Acrylamidopropyltrimonium Chloride / Acrylamide Copolymer Salcare® SC-60 (Ciba SC) | - | - | - | - | - | - | - | 0,3% |
| Glycol Distearate, Laureth-4, Cocamidopropyl Betaine Euperlan® PK-3000 AM (Cognis) | 1,5% | 1,5% | 1,5% | 1,5% | 1,5% | 1,5% | 1,5% | 1,5% |

| | | | | | | | | |
|---|---|---|---|---|---|---|---|---|
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel-douche

| | 41 | 42 | 43 | 44 | 45 | 46 | 47 | 48 |
|---|---|---|---|---|---|---|---|---|
| Sodium Lauryl Ether Sulfate (2OE) Empicol® ESB-3M (Huntsman) | - | 10% | 6% | - | 12% | 12% | - | 14% |
| Ammonium Lauryl Sulfate Rhodapon® L-22 (Rhodia) | 5% | - | 6% | 7% | - | - | 5% | - |
| Ammonium Lauryl Ether Sulfate (2OE) Rhodapex® EA-2 (Rhodia) | - | - | - | 7% | - | - | 7% | - |
| Disodium Cocoamphodiacetate Miranol® C2M Conc NP (Rhodia) | 3% | - | - | - | - | 3% | - | 1% |
| Cocamidopropyl Betaine Mirataine® BET C-30 (Rhodia) | - | 4% | - | - | 3% | - | 2% | 2% |
| Disodium Laureth Sulfosuccinate Geropon® SBFA-30 (Rhodia) | 6% | - | 2% | - | 2% | - | - | - |
| Sodium Lauroyl Glutamate Protelan® AGL-95 (Zschimmer & Schwarz) | - | 2% | - | - | - | - | - | 2% |
| Coco Glucoside Plantacare® 818 UP (Cognis) | 2% | - | - | 1% | - | 2% | - | - |
| Cocamide MIPA Empilan® CIS (Huntsman) | 1% | - | 1,5% | - | 1% | - | 1% | 1% |
| Laureth-2 Empilan® KBE-2 (Huntsman) | - | 1% | - | - | - | - | - | - |
| Sodium Lauroyl Sarcosinate Protelan® LS-9011 (Zschimmer & Schwarz) | - | - | - | 1% | - | - | 2% | - |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Hydroxide de Sodium | 0,2% | 0,2% | 0,2% | 0,2% | - | 0,2% | 0,2% | - |
| Chlorure de Sodium | 0,1% | 0,1% | 0,2% | 0,1% | 1,6% | 0,3% | 0,1% | 1,2% |
| Acrylate/$C_{10-30}$ Alkyl Acrylate Crosspolymer | 1% | 1% | 1% | 1% | - | - | - | - |

| | | | | | | | | |
|---|---|---|---|---|---|---|---|---|
| Carbopol® ETD-2020 (Noveon) | | | | | | | | |
| Acrylate Copolymer Carbopol® Aqua SF-1 (Noveon) | - | - | - | - | 1,5% | 1,5% | 1,5% | 1,5% |
| Glycol Distearate, Laureth-4, Cocamidopropyl Betaine Euperlan® PK-3000 AM (Cognis) | - | - | - | 1,5% | - | 1,5% | - | 1,5% |
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel-douche

| | 57 | 58 | 59 | 60 | 61 |
|---|---|---|---|---|---|
| Sodium Lauryl Ether Sulfate (2OE) Empicol® ESB-3M (Huntsman) | 10% | 10% | 14% | 10% | 12% |
| Cocamidopropyl Betaine Mirataine® BET C-30 (Rhodia) | 2% | 2% | 2% | 2% | 3% |
| Emulsion 0,6µm de Dimethicone: Mirasil® DM-500,000 (Rhodia) | 1% | 1% | 1% | 1% | 1% |
| Polymère A | 0,2% | 0,3% | 0,3% | 0,2% | 0,3% |
| Chlorure de Sodium | 0% | 0% | 0,5% | 0,1% | 0% |
| Hydroxyde de sodium | 0,2% | 0,2% | 0,2% | 0,2% | 0,2% |
| Acrylate/$C_{10-30}$ Alkyl Acrylate Crosspolymer Carbopol® ETD-2020 (Noveon) | 1% | 1% | - | - | - |
| Acrylate Copolymer Carbopol® Aqua SF-1 (Noveon) | - | - | 1,5% | 1,5% | - |
| Carbomer Carbopol® 980 (Noveon) | - | - | - | - | 1,2% |
| Glycerine | - | 1% | 0,5% | - | 0,5% |
| Sodium Laureth Sulfate, Glycol Distearate Cocamide MEA, Laureth-10 Euperlan PK-771 BENZ (Cognis) | 1,5% | - | 1,5% | - | 1,5% |
| PEG-3 Distearate Genapol TS (Clariant) | - | 1.5 % | - | 1.5 % | - |
| Acide citrique | qs | qs | qs | qs | qs |
| Parfum, Conservateurs | qs | qs | qs | qs | qs |
| Eau déminéralisée | qsp 100% | qsp 100% | qsp 100% | qsp 100% | qsp 100% |

Shampooing et/ou gel-douche

| | 62 | 63 | 64 | 65 | 66 | 67 | 68 | 69 |
|---|---|---|---|---|---|---|---|---|
| Sodium Lauryl Ether Sulfate (2OE) Empicol® ESB-3M (Huntsman) | 14% | 10% | 12% | 10% | 14% | 10% | 12% | 10% |
| Coco Betaine Mirataine® BB-FLA (Rhodia) | 2% | 3% | 4% | - | 2% | 3% | 4% | - |
| Disodium Cocoamphodiacetate Miranol® C2M Conc NP (Rhodia) | - | - | - | 2% | - | - | - | 2% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | | 0,5% | | 1,8% | | 0,5% | | 1,8% |
| Hydroxyde de sodium | - | - | - | - | - | - | - | - |
| PEG-200 Hydrogenated Glyceryl Palmate Rewoderm® LI 520-70 (Degussa) | 0,7% | - | - | - | 0,7% | - | - | - |
| PEG-150 Distearate Rewopal® PEG-6000 DS (Degussa) | - | - | 1% | - | - | - | 1% | - |
| Xanthan gum Rhodicare® XC (Rhodia) | - | 0,6% | - | - | - | 0,6% | - | - |
| Hydroxyethyl cellulose Natrosol® 250-HHR HEC (Aqualon) | - | - | - | - | - | - | - | - |
| Hydroxypropyl Guar Jaguar® HP-105 (Rhodia) | - | - | - | 0,8% | - | - | - | 0,8% |
| Propylene Glycol | 0,3% | - | 0,4% | 0,3% | 0,3% | - | 0,4% | 0,3% |
| Sodium Laureth Sulfate, Glycol Distearate Cocamide MEA, Laureth-10 Euperlan PK-771 BENZ (Cognis) | 1,0 % | 1,0% | 1,0% | 1,0% | - | - | - | - |
| PEG-3 Distearate Genapol TS (Clariant) | - | - | - | - | 1.5 % | 1.5 % | 1.5% | 1.5% |
| Acide citrique | qs | qs | qs | qs | qs | qs | qs | qs |
| Parfum, Conservateurs | qs | qs | qs | qs | qs | qs | qs | qs |

| | | | | | | | | |
|---|---|---|---|---|---|---|---|---|
| Eau déminéralisée | qsp 100% | qsp 100% | qsp 100% | qsp 100% | qsp 100% | qsp 100% | qsp 100% | qsp 100% |

Shampooing et/ou gel douche

| | 70 | 71 | 72 | 73 | 74 | 75 | 76 | 77 |
|---|---|---|---|---|---|---|---|---|
| Sodium Lauryl Ether Sulfate (2OE) Empicol® ESB-3M (Huntsman) | 14% | 12% | 10% | 10% | 14% | 12% | 10% | 10% |
| Disodium Cocoamphodiacetate Miranol® C2M Conc NP (Rhodia) | 2% | 4% | 2% | 2% | 2% | 4% | 2% | 2% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | 0,2% | 0,15% | 0,15% | 0,15% | 0,2% | 0,15% | 0,15% | 0,15% |
| Hydroxyde de sodium | 0,2% | 0,2% | 0,2% | 0,2% | 0,2% | 0,2% | 0,2% | 0,2% |
| Acrylate/$C_{10-30}$ Alkyl Acrylate Crosspolymer Carbopol® ETD-2020 (Noveon) | 1% | 1% | 1% | 1% | 1% | 1% | 1% | 1% |
| Guar Hydroxypropyltrimonium Chloride Jaguar® C-13S (Rhodia) | 0,1% | - | - | - | 0,1% | - | - | - |
| Guar Hydroxypropyltrimonium Chloride Jaguar® Excel (Rhodia) | - | - | - | - | - | - | - | - |
| Hydroxypropyl Guar Hydroxypropyltrimonium Chloride Jaguar® C-162 (Rhodia) | - | - | - | - | - | - | - | - |
| Polyquaternium-10 Ucare Polymer® JR-400 (Amerchol) | - | 0,3% | - | - | - | 0,3% | - | - |
| Polyquaternium-7 Merquat® 550 (Nalco) | - | - | - | - | - | - | - | - |
| Polyquaternium-11 | - | - | 0,05% | - | - | - | 0,05% | - |

| | | | | | | | | |
|---|---|---|---|---|---|---|---|---|
| Mirapol® PQ-11 (Rhodia) | | | | | | | | |
| Polyquaternium-22 Merquat® 280 (Nalco) | - | - | - | 0,1% | - | - | - | 0,1% |
| Sodium Laureth Sulfate, Glycol Distearate Cocamide MEA, Laureth-10 Euperlan PK-771 BENZ (Cognis) | 1,5% | 1,5% | 1,5% | 1,5% | - | - | - | - |
| PEG-3 Distearate Genapol TS (Clariant) | - | - | - | - | 1,5% | 1,5% | 1,5% | 1,5% |
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel-douche

| | 78 | 79 | 80 | 81 | 82 | 83 | 84 | 85 |
|---|---|---|---|---|---|---|---|---|
| Tensioactif anionique : Sodium Lauryl Ether Sulfate (2OE) Empicol® ESB-3M (Huntsman) | 14% | 11% | 10% | 10% | 12% | 12% | 10% | 10% |
| Cocamidopropyl Betaine Mirataine® BET C-30 (Rhodia) | 2% | - | 3% | - | - | 4% | 3% | 3% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | 0,1% | 0,1% | 0,1% | - | 0,1% | - | - | - |
| Acrylate Copolymer Carbopol® Aqua SF-1 (Noveon) | 1,5% | 1,5% | 1,5% | 1,5% | 1,5% | 1,5% | 1,5% | 1,5% |
| Hydroxyde de sodium | 0,2% | 0,2% | 0,2% | 0,2% | 0,2% | 0,2% | 0,2% | 0,2% |
| Guar Hydroxypropyltrimonium Chloride Jaguar® C-13S (Rhodia) | 0,1% | - | - | 0,1% | - | - | - | - |
| Polyquaternium-10 Ucare Polymer® JR-400 (Amerchol) | - | 0,3% | - | - | - | - | - | - |
| Polyquaternium-39 Merquat® Plus 3330 (Nalco) | - | - | 0,3% | - | - | - | - | - |
| Polyquaternium-44 Luviquat® UltraCare (BASF) | - | - | - | 0,1% | 0,1% | - | - | - |
| Polyquaternium-67 SoftCAT® Polymer SL (Amerchol) | - | - | - | - | - | 0,2% | - | - |
| Polymethacrylamidopropyltrimonium Chloride Polycare 133 (Rhodia) | - | - | - | - | - | - | 0,2% | |
| Acrylamidopropyltrimonium Chloride / Acrylamide Copolymer Salcare® SC-60 (Ciba SC) | - | - | - | - | - | - | - | 0,3% |
| Sodium Laureth Sulfate, Glycol distearate Cocamide MEA, Laureth-10 Euperlan PK-771 BENZ (Cognis) | 1,5% | 1,5% | 1,5% | 1,5% | 1,5% | 1,5% | 1,5% | 1,5% |

| | | | | | | | | |
|---|---|---|---|---|---|---|---|---|
| Acide citrique | qs | qs | qs | qs | qs | qs | qs | qs |
| Parfum, Conservateurs | qs | qs | qs | qs | qs | qs | qs | qs |
| Eau déminéralisée | qsp 100% | qsp 100% | qsp 100% | qsp 100% | qsp 100% | qsp 100% | qsp 100% | qsp 100% |

Shampooing et/ou gel-douche

| | 86 | 87 | 88 | 89 | 90 | 91 | 92 | 93 |
|---|---|---|---|---|---|---|---|---|
| Tensioactif anionique : Sodium Lauryl Ether Sulfate (2OE) Empicol® ESB-3M (Huntsman) | 14% | 11% | 10% | 10% | 12% | 12% | 10% | 10% |
| Cocamidopropyl Betaine Mirataine® BET C-30 (Rhodia) | 2% | - | 3% | - | - | 4% | 3% | 3% |
| Emulsion 0,6µm de Dimethicone: Mirasil® DM-500,000 (Rhodia) | 1% | 1% | 1% | 1% | 1% | 1% | 1% | 1% |
| Polymère A | 0,2% | 0,2% | 0,2% | 0,2% | 0,2% | 0,2% | 0,2% | 0,2% |
| Chlorure de Sodium | 0,1% | 0,1% | 0,1% | - | 0,1% | - | - | - |
| Acrylate Copolymer Carbopol® Aqua SF-1 (Noveon) | 1,5% | 1,5% | 1,5% | 1,5% | 1,5% | 1,5% | 1,5% | 1,5% |
| Hydroxyde de sodium | 0,2% | 0,2% | 0,2% | 0,2% | 0,2% | 0,2% | 0,2% | 0,2% |
| Guar Hydroxypropyltrimonium Chloride Jaguar® C-13S (Rhodia) | 0,1% | - | - | 0,1% | - | - | - | - |
| Polyquaternium-10 Ucare Polymer® JR-400 (Amerchol) | - | 0,3% | - | - | - | - | - | - |
| Polyquaternium-39 Merquat® Plus 3330 (Nalco) | - | - | 0,3% | - | - | - | - | - |
| Polyquaternium-44 Luviquat® UltraCare (BASF) | - | - | - | 0,1% | 0,1% | - | - | - |
| Polyquaternium-67 SoftCAT® Polymer SL (Amerchol) | - | - | - | - | - | 0,2% | - | - |
| Polymethacrylamidopropyltrimonium Chloride Polycare 133 (Rhodia) | - | - | - | - | - | - | 0,2% | |
| Acrylamidopropyltrimonium Chloride / Acrylamide Copolymer Salcare® SC-60 (Ciba SC) | - | - | - | - | - | - | - | 0,3% |
| PEG-3 Distearate Genapol TS (Clariant) | 1,5% | 1,5% | 1,5% | 1,5% | 1,5% | 1,5% | 1,5% | 1,5% |
| Acide citrique | qs | qs | qs | qs | qs | qs | qs | qs |

| | | | | | | | | |
|---|---|---|---|---|---|---|---|---|
| Parfum, Conservateurs | qs | qs | qs | qs | qs | qs | qs | qs |
| *Eau déminéralisée* | qsp 100% | qsp 100% | qsp 100% | qsp 100% | qsp 100% | qsp 100% | qsp 100% | qsp 100% |

Shampooing et/ou gel-douche

| | 100 | 101 | 102 | 103 | 104 | 105 |
|---|---|---|---|---|---|---|
| Sodium Lauryl Ether Sulfate (2OE) Empicol® ESB-3M (Huntsman) | - | 12% | 14% | - | 12% | 14% |
| Ammonium Lauryl Sulfate Rhodapon® L-22 (Rhodia) | 7% | - | - | 7% | - | - |
| Ammonium Lauryl Ether Sulfate (2OE) Rhodapex® EA-2 (Rhodia) | 7% | - | - | 7% | - | - |
| Disodium Cocoamphodiacetate Miranol® C2M Conc NP (Rhodia) | - | 3% | 1% | - | 3% | 1% |
| Cocamidopropyl Betaine Mirataine® BET C-30 (Rhodia) | - | - | 2% | - | - | 2% |
| Disodium Laureth Sulfosuccinate Geropon® SBFA-30 (Rhodia) | - | - | - | - | - | - |
| Sodium Lauroyl Glutamate Protelan® AGL-95 (Zschimmer & Schwarz) | - | - | 2% | - | - | 2% |
| Coco Glucoside Plantacare® 818 UP (Cognis) | 1% | 2% | - | 1% | 2% | - |
| Cocamide MIPA Empilan® CIS (Huntsman) | - | - | 1% | - | - | 1% |
| Laureth-2 Empilan® KBE-2 (Huntsman) | - | - | - | - | - | - |
| Sodium Lauroyl Sarcosinate Protelan® LS-9011 (Zschimmer & Schwarz) | 1% | - | - | 1% | - | - |
| Emulsion 0,6µm de Dimethicone: Mirasil® DM-500,000 (Rhodia) | 1% | 1% | 1% | 1% | 1% | 1% |
| Polymère A | 0,2% | 0,2% | 0,2% | 0,2% | 0,2% | 0,2% |
| Hydroxide de Sodium | 0,2% | 0,2% | - | 0,2% | 0,2% | - |
| Chlorure de Sodium | 0,1% | 0,3% | 1,2% | 0,1% | 0,3% | 1,2% |
| Acrylate/$C_{10-30}$ Alkyl Acrylate Crosspolymer Carbopol® ETD-2020 (Noveon) | 1% | - | - | 1% | - | - |

| | | | | | | |
|---|---|---|---|---|---|---|
| Acrylate Copolymer Carbopol® Aqua SF-1 (Noveon) | - | 1,5% | 1,5% | - | 1,5% | 1,5% |
| Sodium Laureth Sulfate, Glycol Distearate Cocamide MEA, Laureth-10 Euperlan PK-771 BENZ (Cognis) | 1,5% | 1,5% | 1,5% | - | - | - |
| PEG-3 Distearate Genapol TS (Clariant) | - | - | - | 1,5% | 1,5% | 1,5% |
| Acide citrique | qs | qs | qs | qs | qs | qs |
| Parfum, Conservateurs | qs | qs | qs | qs | qs | qs |
| Eau déminéralisée | qsp 100% | qsp 100% | qsp 100% | qsp 100% | qsp 100% | qsp 100% |

## Revendications

**1.** Ingrédient concentré pour le traitement et/ou la modification de surfaces, comprenant les produits suivants:

a) de 10 à 75% en poids d'un agent conditionneur choisi parmi les polyorganosiloxanes,
b) de 0,5 à 20% en poids d'un polymère d'aide au dépôt,
c) de 0 à 15% en poids d'un tensioactif, et
d) de l'eau,

**caractérisé en ce qu'**il est sous forme d'une émulsion directe comprenant des gouttelettes de l'agent conditionneur a) dispersées dans l'eau, et **en ce que**

- le rapport en poids entre le produit c), s'il est présent, et le produit a) est inférieur à 1, de préférence inférieur à 0,5, de préférence inférieur à 0,1, et
- ledit polymère d'aide au dépôt étant un copolymère ampholyte comprenant :

- 0,1 à 50% en nombre d'unités $B_{CAT}$, dérivant de la polymérisation d'au moins un composé monomère $B_{CAT}$ de formule générale I :

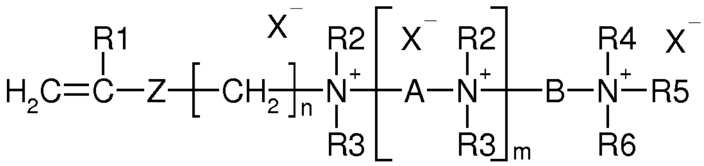

dans laquelle:

- $R_1$ est un atome d'hydrogène, un groupe méthyle ou éthyle ;
- $R_2$, $R_3$, $R_4$, $R_5$, $R_6$, identiques ou différents, sont des groupes alkyle, hydroxyalkyle ou aminoalkyle, linéaires ou ramifiés, en $C_1$-$C_6$, de préférence en$C_1$-$C_4$,
- m est un nombre entier de 0 à 10, de préférence de 0 à 2 ;
- n est un nombre entier de 1 à 6, de préférence 2 à 4 ;
- Z représente un groupe -C(O)O-, -C(O)NH- ou un atome d'oxygène ;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles ;
- $X^-$, identiques ou différents, représentent des contre-ions ;
- des unités $B_A$ dérivant de la polymérisation d'au moins un monomère $B_A$ hydrophile portant une fonction à caractère acide copolymérisable avec $B_A$, anionique ou susceptible d'être anionique,
- éventuellement des unités $B_N$ dérivant d'au moins un monomère $B_N$ à insaturation éthylénique, de charge neutre, copolymérisable avec $B_{CAT}$ et $B_A$, de préférence un composé monomère hydrophile à insaturation éthylénique de charge neutre portant un ou plusieurs groupes hydrophiles, copolymérisable avec $B_{CAT}$ et $B_A$,
- la quantité d'unités $B_A$ et éventuellement $B_N$ étant de 50 à 99,9 % en nombre.

**2.** Ingrédient selon la revendication précédente, **caractérisé en ce que** la quantité totale en poids en produits a) et b) dans l'ingrédient est d'au moins 20%, de préférence d'au moins 50%, de préférence d'au moins 60%.

**3.** Ingrédient selon la revendication 1 ou 2, **caractérisé en ce que** la quantité d'eau en poids est inférieure à 90% en poids, de préférence inférieure à 75% en poids.

**4.** Ingrédient selon l'une des revendications précédentes, **caractérisé en ce que** le rapport en poids entre la produit b) et le produit a) dans l'ingrédient concentré est de préférence compris entre 0,05 et 9, de préférence entre 0,05 et 0,5, de préférence entre 0,075 et 0,3.

**5.** Ingrédient selon l'une des revendications précédentes, **caractérisé en ce qu'**il comprend de 20 à 70 % en poids de produit a).

**6.** Ingrédient selon l'une des revendications précédentes, **caractérisé en ce qu'**il comprend de 1 à 15% en poids de produit b).

**7.** Ingrédient selon l'une des revendications précédentes, **caractérisé en ce que** l'émulsion est une émulsion dont la taille moyenne des gouttelettes est supérieure ou égale à 2 $\mu$m, ou dont la taille moyenne des gouttelettes est comprise entre 0,15 $\mu$m et 2 $\mu$m, ou dont la taille moyenne des gouttelettes est inférieure ou égale à 0,15 $\mu$m.

**8.** Ingrédient selon l'une des revendications précédentes **caractérisé en ce que** le polyorganosiloxane a) est un polydiméthylorganosiloxanesiloxane, ou un polyorganosiloxane présentant des groupes amine, des groupes ammonium quaternaire, des groupes hydroxyle, des groupes polyoxyalkylène, des groupes aromatiques, ou plusieurs de ces groupes.

**9.** Ingrédient selon l'une quelconque des revendications 1 à 8, **caractérisé en ce que**:

- les unités $B_{CAT}$ dérivent de monomère $B_{CAT}$ de formule suivante:

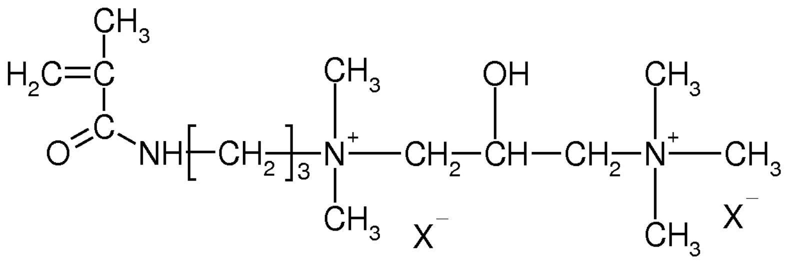

$X^-$ représentant l'ion chlorure ou méthyl sulfate,
- les unités $B_A$ dérivent d'acide acrylique,
- le polymère ne comprend pas d'unités $B_N$,
- le rapport en nombre entre les unités $B_A$ et les unités $B_{CAT}$ est de 50/50 à 90/10.

**10.** Ingrédient selon l'une des revendications précédentes, **caractérisé en ce que** le tensioactif c) est un tensioactif non ionique, de préférence en alcool ethoxylé.

**11.** Utilisation de l'ingrédient selon l'une des revendications précédentes, dans une composition cosmétique.

**12.** Utilisation selon la revendication précédente, **caractérisé en ce que** la composition cosmétique comprend:

- un vecteur cosmétiquement acceptable, de préférence aqueux,
- éventuellement au moins un tensioactif, et
- l'ingrédient concentré.

**13.** Utilisation selon la revendication précédente, **caractérisée en ce que** elle comprend:

- le tensioactif en proportion en poids dans la composition comprise entre 0 et 30% de préférence entre, 5 et 30% en poids, le tensioactif comprenant un tensioactif anionique et éventuellement un tensioactif amphotère,
- le polymère d'aide au dépôt en proportion en poids dans la composition comprise entre 0,01 et 5%, de préférence entre 0,05 et 1,5%, de préférence de 0,1 à 0,3%.

**14.** Utilisation selon l'une des revendications 12 ou 13, **caractérisée en ce que** la composition est un shampoing comprenant entre 10 et 20% de tensioactif, ou un gel-douche comprenant entre 5 et 15% de tensioactif, ou un après shampoing comprenant moins de 5% de tensioactif.

**Patentansprüche**

**1.** Konzentrierter Inhaltsstoff zur Behandlung und/oder Modifikation von Oberflächen, der die folgenden Produkte umfasst:

a) 10 bis 75 Gew.-% eines Konditionierungsmittels, ausgewählt aus Polyorganosiloxanen,
b) 0,5 bis 20 Gew.-% eines Ablagerungshilfsmittel-Polymers,
c) 0 bis 15 Gew.-% eines Tensids und
d) Wasser,

**dadurch gekennzeichnet, dass** er in Form einer direkten Emulsion vorliegt, die in Wasser dispergierte Tröpfchen des Konditionierungsmittels a) umfasst, und dadurch, dass

- das Gewichtsverhältnis zwischen dem Produkt c), wenn vorhanden, und dem Produkt a) kleiner als 1, vorzugsweise kleiner als 0,5, bevorzugt kleiner als 0,1 ist und
- das Ablagerungshilfsmittel-Polymer ein ampholytisches Copolymer ist, das Folgendes umfasst:

  - 0,1 bis 50%, bezogen auf die Anzahl, Einheiten $B_{CAT}$, die aus der Polymerisation mindestens einer

Monomerverbindung $B_{CAT}$ der allgemeinen Formel I stammen:

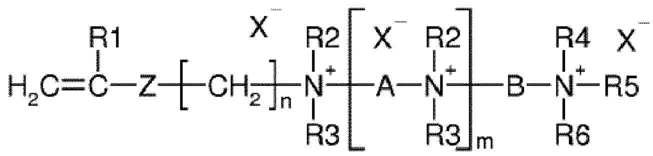

,

worin:

- $R_1$ ein Wasserstoffatom, eine Methyl- oder Ethylgruppe ist;
- $R_2$, $R_3$, $R_4$, $R_5$, $R_6$, die gleich oder verschieden sind, gerade oder verzweigte $C_1$-$C_6$-, vorzugsweise $C_1$-$C_4$-Alkyl-, -Hydroxyalkyl- oder -Aminoalkylgruppen sind,
- m eine ganze Zahl von 0 bis 10, vorzugsweise von 0 bis 2 ist;
- n eine ganze Zahl von 1 bis 6, vorzugsweise von 2 bis 4 ist;
- Z eine Gruppe -C(O)O-, -C(O)NH- oder ein Sauerstoffatom darstellt;
- A eine Gruppe $(CH_2)p$ darstellt, wobei p eine ganze Zahl von 1 bis 6, vorzugsweise von 2 bis 4 ist;
- B eine gerade oder verzweigte $C_2$-$C_{12}$-, vorzugsweise $C_3$-$C_6$-Polymethylenkette darstellt, die gegebenenfalls durch ein oder mehrere Heteroatome oder Heterogruppe, insbesondere 0 oder NH, unterbrochen und gegebenenfalls mit einer oder mehreren Hydroxyl- oder Amino-, vorzugsweise Hydroxylgruppen substituiert ist,
- $X^-$, die gleich oder verschieden sind, Gegenionen darstellen;

- Einheiten $B_A$, die aus der Polymerisation mindestens eines hydrophilen, anionischen oder potenziell anionischen Monomers $B_A$ stammen, das eine mit $B_A$ copolymerisierbare Funktion mit saurem Charakter trägt,
- gegebenenfalls Einheiten $B_N$, die von mindestens einem ethylenisch ungesättigten Monomer $B_N$ mit neutraler Ladung stammen, das mit $B_{CAT}$ und $B_A$ copolymerisierbar ist, vorzugsweise einer mit $B_{CAT}$ und $B_A$ copolymerisierbaren hydrophilen Monomerverbindung mit ethylenischer Ungesättigtheit und neutraler Ladung, die eine oder mehrere hydrophile Gruppen trägt,
- wobei Menge an Einheiten $B_A$ und gegebenenfalls $B_N$, bezogen auf die Anzahl, von 50 bis 99,9% beträgt.

2. Inhaltsstoff nach dem vorhergehenden Anspruch, **dadurch gekennzeichnet, dass** die Gesamtmenge, bezogen auf Gewicht, an Produkten a) und b) in dem Inhaltsstoff mindestens 20%, vorzugsweise mindestens 50%, bevorzugt mindestens 60% beträgt.

3. Inhaltsstoff nach Anspruch 1 oder 2, **dadurch gekennzeichnet, dass** die Menge an Wasser, bezogen auf Gewicht, weniger als 90 Gew.-%, vorzugsweise weniger als 75 Gew.-% beträgt.

4. Inhaltsstoff nach einem der vorhergehenden Ansprüche, **dadurch gekennzeichnet, dass** das Gewichtsverhältnis zwischen dem Produkt b) und dem Produkt a) in dem konzentrierten Inhaltsstoff vorzugsweise zwischen 0,05 und 9, vorzugsweise zwischen 0,05 und 0,5, bevorzugt zwischen 0,075 und 0,3 beträgt.

5. Inhaltsstoff nach einem der vorhergehenden Ansprüche, **dadurch gekennzeichnet, dass** er 20 bis 70 Gew.-% von Produkt a) umfasst.

6. Inhaltsstoff nach einem der vorhergehenden Ansprüche, **dadurch gekennzeichnet, dass** er 1 bis 15 Gew.-% von Produkt b) umfasst.

7. Inhaltsstoff nach einem der vorhergehenden Ansprüche, **dadurch gekennzeichnet, dass** es sich bei der Emulsion um eine Emulsion handelt, deren mittlere Tröpfchengröße größer als oder gleich 2 $\mu$m ist oder deren mittlere Tröpfchengröße zwischen 0,15 $\mu$m und 2 $\mu$m beträgt oder deren mittlere Tröpfchengröße kleiner als oder gleich 0,15 $\mu$m ist.

8. Inhaltsstoff nach einem der vorhergehenden Ansprüche, **dadurch gekennzeichnet, dass** das Polyorganosiloxan a) ein Polydimethylorganosiloxansiloxan oder ein Polyorganosiloxan ist, das Amingruppen, quaternäre Ammoniumgruppen, Hydroxylgruppen, Polyoxyalkylengruppen, aromatische Gruppen oder mehrere dieser Gruppen aufweist.

**9.** Inhaltsstoff nach einem der Ansprüche 1 bis 8, **dadurch gekennzeichnet, dass**

- die Einheiten $B_{CAT}$ von dem Monomer $B_{CAT}$ der folgenden Formel stammen:

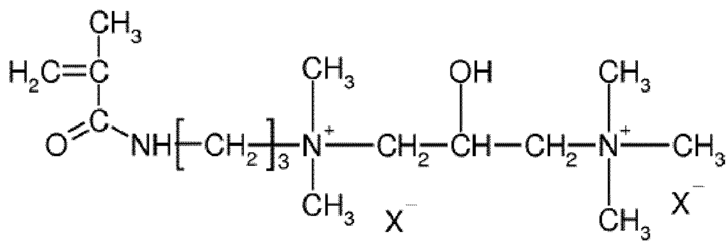

,

wobei $X^-$ ein Chlorid- oder Methylsulfation darstellt,
- die Einheiten $B_A$ von Acrylsäure stammen,
- das Polymer keine Einheiten $B_N$ enthält.
- das Verhältnis zwischen den Einheiten $B_A$ und den Einheiten $B_{CAT}$, bezogen auf die Anzahl, von 50/50 bis 90/10 beträgt.

**10.** Inhaltsstoff nach einem der vorhergehenden Ansprüche, **dadurch gekennzeichnet, dass** das Tensid c) ein nichtionisches Tensid, vorzugsweise ethoxylierter Alkohol ist.

**11.** Verwendung des Inhaltsstoffs nach einem der vorhergehenden Ansprüche in einer Kosmetikzusammensetzung.

**12.** Verwendung nach dem vorhergehenden Anspruch, **dadurch gekennzeichnet, dass** die Kosmetikzusammensetzung Folgendes umfasst:

- ein kosmetisch annehmbares, vorzugsweise wässriges Vehikel,
- gegebenenfalls mindestens ein Tensid und
- den konzentrierten Inhaltsstoff.

**13.** Verwendung nach dem vorhergehenden Anspruch, **dadurch gekennzeichnet, dass** sie Folgendes umfasst:

- das Tensid in einem Gewichtsanteil in der Zusammensetzung von zwischen 0 und 30 Gew.-%, vorzugsweise zwischen 5 und 30 Gew.-%, wobei das Tensid ein anionisches Tensid und gegebenenfalls ein amphoteres Tensid umfasst,
- das Ablagerungshilfsmittel-Polymer in einem Gewichtsanteil in der Zusammensetzung von zwischen 0,01% und 5%, vorzugsweise zwischen 0,05 und 1,5%, bevorzugt von 0,1% bis 0,3%.

**14.** Inhaltsstoff nach einem der Ansprüche 12 oder 13, **dadurch gekennzeichnet, dass** die Kosmetikzusammensetzung ein Shampoo ist, das zwischen 10 und 20% Tensid umfasst, oder ein Duschgel, das zwischen 5 und 15% Tensid umfasst, oder eine Haarspülung, die weniger als 5% Tensid umfasst.

## Claims

**1.** Concentrated ingredient for treating and/or modifying surfaces, comprising the following products:

a) from 10% to 75% by weight of a conditioning agent chosen from polyorganosiloxanes,
b) from 0.5% to 20% by weight of a polymer for aiding deposition,
c) from 0 to 15% by weight of a surfactant, and
d) water,

**characterized in that** it is in the form of a direct emulsion comprising droplets of the conditioning agent a) dispersed in water, and **in that**

- the weight ratio between product c), if it is present, and product a) is less than 1, preferably less than 0.5 and preferably less than 0.1, and
- said polymer for aiding deposition being an ampholytic copolymer comprising:

  - 0.1% to 50% by number of units $B_{CAT}$ derived from the polymerization of at least one monomer compound $B_{CAT}$ of general formula I:

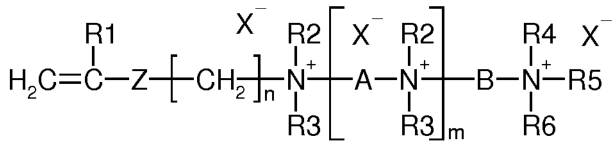

  in which:

    - $R_1$ is a hydrogen atom or a methyl or ethyl group;
    - $R_2$, $R_3$, $R_4$, $R_5$ and $R_6$, which may be identical or different, are linear or branched $C_1$-$C_6$ and preferably $C_1$-$C_4$ alkyl, hydroxyalkyl or aminoalkyl groups,
    - m is an integer from 0 to 10 and preferably from 0 to 2;
    - n is an integer from 1 to 6 and preferably from 2 to 4;
    - Z represents a -C(O)O- or -C(O)NH- group or an oxygen atom;
    - A represents a group $(CH_2)_p$, p being an integer from 1 to 6 and preferably from 2 to 4;
    - B represents a linear or branched $C_2$-$C_{12}$ and advantageously $C_3$-$C_6$ polymethylene chain, optionally interrupted with one or more heteroatoms or hetero groups, especially 0 or NH, and optionally substituted with one or more hydroxyl or amino groups, preferably hydroxyl groups;
    - $X^-$, which may be identical or different, represent counterions;

  - units $B_A$ derived from the polymerization of at least one hydrophilic monomer $B_A$ bearing a function of acidic nature that is copolymerizable with $B_A$, which is anionic or potentially anionic,
  - optionally units $B_N$ derived from at least one ethylenically unsaturated monomer $B_N$ of neutral charge, which is copolymerizable with $B_{CAT}$ and $B_A$, preferably an ethylenically unsaturated hydrophilic monomer compound of neutral charge bearing one or more hydrophilic groups, which is copolymerizable with $B_{CAT}$ and $B_A$,
  - the amount of units $B_A$ and optionally $B_N$ being from 50% to 99.9% by number.

2. Ingredient according to the preceding claim, **characterized in that** the total weight amount of products a) and b) in the ingredient is at least 20%, preferably at least 50% and preferably at least 60%.

3. Ingredient according to Claim 1 or 2, **characterized in that** the weight amount of water is less than 90% by weight and preferably less than 75% by weight.

4. Ingredient according to one of the preceding claims, **characterized in that** the weight ratio between product b) and product a) in the concentrated ingredient is preferably between 0.05 and 9, preferably between 0.05 and 0.5 and preferably between 0.075 and 0.3.

5. Ingredient according to one of the preceding claims, **characterized in that** it comprises from 20% to 70% by weight of product a).

6. Ingredient according to one of the preceding claims, **characterized in that** it comprises from 1% to 15% by weight of product b).

7. Ingredient according to one of the preceding claims, **characterized in that** the emulsion is an emulsion whose mean droplet size is greater than or equal to 2 μm, or whose mean droplet size is between 0.15 μm and 2 μm, or whose mean droplet size is less than or equal to 0.15 μm.

8. Ingredient according to one of the preceding claims, **characterized in that** the polyorganosiloxane a) is a polydimethylorganosiloxanesiloxane, or a polyorganosiloxane containing amine groups, quaternary ammonium groups, hydroxyl groups, polyoxyalkylene groups, aromatic groups, or several of these groups.

**9.** Ingredient according to any one of Claims 1 to 8, **characterized in that**:

- the units $B_{CAT}$ are derived from the monomer $B_{CAT}$ having the following formula:

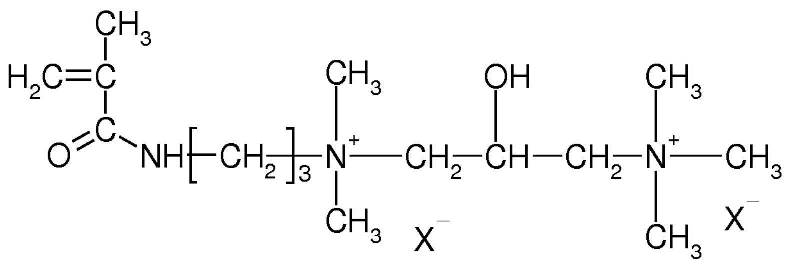

$X^-$ representing a chloride or methyl sulfate ion,
- the units $B_A$ are derived from acrylic acid,
- the polymer does not comprise any units $B_N$,
- the numerical ratio between the units $B_A$ and the units $B_{CAT}$ is from 50/50 to 90/10.

**10.** Ingredient according to one of the preceding claims, **characterized in that** the surfactant c) is a nonionic surfactant, preferably an ethoxylated alcohol.

**11.** Use of the ingredient according to one of the preceding claims, in a cosmetic composition.

**12.** Use according to the preceding claim, **characterized in that** the cosmetic composition comprises:

- a cosmetically acceptable vector, which is preferably aqueous,
- optionally at least one surfactant, and
- the concentrated ingredient.

**13.** Use according to the preceding claim, **characterized in that** it comprises:

- the surfactant in a weight proportion in the composition of between 0 and 30% and preferably between 5% and 30% by weight, the surfactant comprising an anionic surfactant and optionally an amphoteric surfactant,
- the polymer for aiding deposition in a weight proportion in the composition of between 0.01% and 5%, preferably between 0.05% and 1.5% and preferably from 0.1% to 0.3%.

**14.** Use according to either of Claims 12 and 13, **characterized in that** the composition is a shampoo comprising between 10% and 20% of surfactant, or a shower gel comprising between 5% and 15% of surfactant, or a hair conditioner comprising less than 5% of surfactant.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 432951 A
- EP 529883 A
- EP 904045 A
- US 20030108504 A
- FR 2796392
- WO 0014053 A
- US 4565647 A
- EP 219048 A
- US 3959230 A
- US 3893929 A
- US 4116896 A
- US 4702857 A
- US 4770666 A
- US 4968451 A
- EP 540374 A
- FR 2728915 A
- FR 2236926 A
- US 4711730 A
- US 4721580 A
- US 4877896 A
- FR 2334698 A
- US 4597898 A
- EP 11984 A
- WO 9216187 A
- US 3308067 A
- EP 66915 A